(19) Europäisches Patentamt
European Patent Office
Office européen des brevets

(11) Publication number: **0 431 648 B1**

(12) # EUROPEAN PATENT SPECIFICATION

(45) Date of publication of patent specification: **22.03.95**

(51) Int. Cl.[6]: **A61L 9/015**, B01D 53/38

(21) Application number: **90123600.0**

(22) Date of filing: **07.12.90**

(54) **Pollution abatement system.**

(30) Priority: **08.12.89 US 447996**

(43) Date of publication of application:
**12.06.91 Bulletin 91/24**

(45) Publication of the grant of the patent:
**22.03.95 Bulletin 95/12**

(84) Designated Contracting States:
**AT BE CH DE DK ES FR GB GR IT LI LU NL SE**

(56) References cited:
**EP-A- 0 282 301**
**EP-A- 0 361 385**
**DE-A- 3 905 842**
**FR-A- 2 581 565**
**GB-A- 2 229 365**
**US-A- 4 343 765**
**US-A- 4 370 301**

**D3: Chemical Abstracts, vol. 109, 1988, page 385, abstr.-no. 155422g**

(73) Proprietor: **UOP**
**25 East Algonquin Road**
**Des Plaines,**
**Illinois 60017-5017 (US)**

(72) Inventor: **Keller II, George Ernest**
**1207 Ellen Drive**
**South Charleston,**
**West Virginia (25303) (US)**
Inventor: **Miller, Jay Fingeret**
**828 Gordon Drive**
**South Charleston,**
**West Virginia (25303) (US)**
Inventor: **Rodberg, Joan Ayer**
**2007 Kanawha Avenue**
**S.E. Charleston,**
**West Virginia (25304) (US)**

(74) Representative: **Weinhold, Peter, Dr. et al**
**Patentanwälte Dipl.-Ing. G. Dannenberg**
**Dr. P. Weinhold**
**Dr. D. Gudel**
**Dipl.-Ing. S. Schubert**
**Dr. P.**
**Barz**
**Siegfriedstrasse 8**
**D-80803 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 431 648 B1

## Description

The present invention relates to a method for the treatment of confined areas utilized for social activities through which forced air is circulated whereby pollutant levels are reduced. The method is especially adapted to treatment of circulated air in office buildings, hospitals, homes, workplaces, clean rooms, and the like facilities, and may be effected while the facilities are being inhabited and/or contain sensitive equipment.

With the oil shortages of the early to mid-1970s came a need to conserve on the use of oil and gas in the heating of structures used for social activities, such as housing people and equipment subject to environmental attack. Housing and office buildings were constructed or modified to utilize the minimum amount of energy to heat and cool them. This was done by sealing the structures from the external environment and relying on recirculation of the already heated or cooled air. Initially, little concern was expressed about the quality of the air in terms of pollutants that were being recirculated. The prime concern has been the energy conservation issue of keeping heated or cool air within the structures so as to minimize the amount of air that had to be heated or cooled. The environmental impact is only recently becoming well documented. The current studies have evolved the term "sick building syndrome" to cover the problem which has been termed "the worst health problem in the United States, accounting for 50 percent of all illnesses, says a study by the Massachusetts Commission on Indoor Air Pollution" JAPCA, vol. 39, No. 7, July 1989, p. 924.

The United States Environmental Protection Agency has been tracking the issues surrounding indoor air quality, and in a report dated June 1987, EPA-600/8-87-031, at page 6, it reported:

"Since indoor air pollution problems are primarily a function of the products and materials used within specific building settings, the character of the problem will be different in each setting and the most appropriate mitigation strategy will depend on a variety of factors."

*Anthony V. Nero, Jr.,* Scientific American, May 1988, Volume 258, Number 5, pp. 42-48, in an article entitled "Controlling Indoor Air Pollution," describes the problem as follows:

.... the greatest exposures to airborne combustion products, volatile toxic chemicals and radioactivity typically occur not outdoors but inside residences, offices and other nomenclatures buildings -- settings that traditionally have been neglected by pollution-control agencies. Indeed, the health risks incurred merely by breathing the air at home can substantially exceed the general limits on risk that regulatory agencies impose in controlling pollutants in outdoor air or drinking water.

.... The causes of indoor air pollution are so diverse and the concentrations are so variable that continual monitoring would be required in virtually all buildings--more than 80 million in the U.S. alone.

Nero found the pollutants in indoor air to be similar to those found outdoors and the pollutants measured in the highest concentrations indoors are those that arise from within buildings or their substructures. He characterized cigarette smoke, which is composed mainly of organic aerosols, or tiny airborne particles; heating and cooking appliances that burn natural gas, kerosene, oil and wood (or peat, coal and dung, as is the case in some countries) emitting varying quantities of respirable particles along with carbon oxides, nitrogen oxides and trace organic chemicals; methylene chloride; formaldehyde; and a vast range of more complex organic chemicals that are given off by building materials, furniture, cleaning fluids, pesticides, paints and paint strippers, products of living organisms or the organisms themselves, such as bacteria, fungi and house mites.

According to Nero, the consequences are spelled out to include a "wide range of health risks."

Cigarette smoke, asbestos fibers, the decay products of radon, formaldehyde and many other organic chemicals are demonstrated or potential carcinogens. Most of these pollutants can also lead to chronic or acute diseases, such as respiratory infections and allergic responses, as can combustion products in general and a variety of indoor bacteria and fungi. Extremely high levels of carbon monoxide -- a combustion product -- can even result in immediate death. Yet only in a relatively few cases, such as acute allergic reactions or carbon monoxide poisoning, is there a clear-cut relation between a given exposure to an indoor pollutant and an associated health effect.

The complexity of the problem is demonstrated in an article by *P. Morey,* Proceeding of the ASHRAE Conference, April 1988, Atlanta, Georgia, entitled: "Microorganisms in Buildings and HVAC Systems: A Summary of 21 Environmental Studies", published by ASHRAE 1988, Atlanta, Ga.

---- Microbiological reservoirs and amplification sites were found in 18 of the 21 buildings. In several buildings, air sampling showed that occupants were exposed to unusual numbers and kinds of microorganisms.

In 11 of 18 buildings, maintenance of mechanical systems was difficult, if not impossible, because systems were improperly designed and inaccessible. Unusual bioaerosol exposures in the occupied space

were shown in several buildings to be due to microbiological contamination of porous man-made insulation present inside air-handling and fan coil units. Contaminated soundliner was present in 9 of 18 problem buildings. Other significant sources of microbiological contamination in buildings included stagnant water in drain pans (10 of 18 buildings), excessive relative humidity (6 of 18 buildings), floods (6 of 18 buildings), and the location of outdoor air intakes near external bioaerosol sources (6 of 18 buildings).

The author's prescription for dealing with the problem included "[T]wo basic types of actions are effective in controlling microbiological contamination" and they are "[R]estricting the availability of moisture is the most direct method for limiting the growth of microorganisms" and "removal of nutrients (carbon sources) needed for amplification." This included "[M]ore efficient filters (McNall 1975) and better housekeeping."

The author found microbiological pollution to create "widespread irritation of the eyes and upper respiratory system, headache, and odor annoyance."

According to ***Godish,*** Indoor Air Pollution Control published by Lewis Publishers, Chelsea, MI 48118, 1989, page 53, in a section dealing with the problems of biogenic particles:

Biogenic particles are particles of biological origin. They include viable entities such as bacteria, fungi, viruses, amoebae, algae,and pollen grains and the no-longer-viable forms of the same. They also include plant parts; insect parts and wastes; animal saliva, urine, and dander; human dander; and a variety of organic dusts.

Exposure to airborne biogenic particles can result in a number of disease or illness syndromes. The relationship between pathogenic disease caused by bacteria and viruses and the airborne transmission of such disease has been known for over a half century. Many communicable diseases are transmitted from one individual to another by the suspension of infective particles in air and their subsequent inhalation by previously unaffected individuals. Influenza, tuberculosis, and colds are notable examples. The close proximity of individuals to each other in buildings results in an increased risk of airborne transmission of infective particles that cause disease.

In general, infectious diseases do not fall within the scope of indoor air quality of building-related health concerns. An exception is Legionnaires' disease, which has been linked to contamination of indoor air by building systems such as cooling towers and evaporative condensers. Airborne biogenic particles are notable because they have been known to cause allergies, asthma, and hypersensitivity pneumonitis/humidifier fever in addition to Legionaires' disease, and are suspected by some investigators to cause subjective symptoms associated with sick building syndrome.

***Lois R. Ember,*** Chemical & Engineering News. 12/5/88, in an article entitled "Survey Finds High Indoor Levels Of Volatile Organic Chemicals," reported:

People are exposed to much higher levels of dangerous air pollutants in office buildings, schools, hospitals, and nursing homes than outdoors, even in heavily industrialized cities, the Environmental Protection Agency says.

A five-year, $850,000 study by EPA, the first of its kind, found more than 500 volatile organic compounds (VOC's), most only once, in a survey of 10 buildings. Indoor concentrations for all compounds except benzene exceeded outdoor levels, clearly indicating indoor sources. Aliphatic hydrocarbons were the compounds found most frequently, followed by aromatic hydrocarbons and chlorinated hydrocarbons.

New buildings were found to have very high concentrations of aromatic and aliphatic hydrocarbons immediately after completion. Over the next several months, concentrations dropped rapidly, sometimes by a order of magnitude. Occasionally it took up to a year for indoor concentrations to drop to the concentration of the same pollutants outdoors. Recognizing this, Scandinavian countries require 100% outdoor air as makeup air for the first six months of a building's existence.

In the 10-building monitoring effort, EPA researchers tracked the VOC's to common items: building materials, cleaning solvents, furnishings, and pesticides. Latex caulk, carpet adhesive, latex paint, telephone cable, carpeting, and particle board were some of the highest emitters of VOC's. With the thrust to tighter, more energy efficient buildings, these compounds can build up to very high concentrations.

Most people spend nearly 90% of their time indoors, and for this reason are potentially at greater risk of developing health problem from breathing indoor air than from outdoor air. And those spending the most time indoors -- the very young, the very old, and the chronically ill -- are potentially the most susceptible to such adverse effects as headaches, depression, fatigue, irritability, ladylike symptoms, heart disease, and cancer.

In some new buildings xylenes and decane, chemicals associated with the so-called 'sick building syndrome', were found in concentrations 100 times greater than outdoor levels. According to Axelrod, the sick building syndrome is 'a collection of symptoms for which a cause has not been identified.' The potential causes include chemicals (very low levels of VOC's) as well as biological agents such as bacteria

and fungi. Many people in sick buildings, including EPA's own headquarters building, claim to have become chemically sensitive, though there is no clinically acceptable test for chemical sensitivity. Many cite allergy-like symptoms, which seems to implicate biological agents.

**Myriad organic compounds typically found indoors**

| | | |
|---|---|---|
| **Aromatic hydrocarbons** | **Alcohols** | 2-Methylhexane |
| | | 3-Methylhexane |
| | 2-Butyloctanol | 3-Methylnonane |
| Benzene | 1-Dodecanol | 2-Methylpentane |
| Diethylbenzenes | 2-Ethyl-1-hexanol | 3-Methylpentane |
| Dimethylethylbenzenes | 1-Hexanol | Nonane |
| Ethylbenzene | | Octane |
| Ethylmethylbenzenes | **Esters** | Pentadecane |
| Methylnaphtalenes | | Pentane |
| 1-Propylbenzene | Ethyl acetate | Tetradecane |
| Naphthalene | 1-Hexyl butanoate | Tridecane |
| Propylmethylbenzenes | | Undecane |
| Styrene | | |
| Toluene | **Aliphatics** | **Aldehydes** |
| Trimethylbenzenes | | |
| Xylenes | Cyclohexane | Decanal |
| | Decane | Nonanal |
| **Halogenated hydrocarbons** | Dodecane | |
| | 2,4-Dimethylhexane | **Miscellaneous** |
| | 1,3-Dimethyl-cyclopentane | |
| Chloroform | | Acetic Acid |
| Dichlorobenzenes | Eicosane | Acetone |
| Dichloromethane | Heptane | Dimethylphenols |
| Tetrachloroethylene | Hexane | Ethylene Oxide |
| 1,1,1-Trichloroethane | Methylcyclohexane | |
| Trichloroethylene | 4-Methyldecane | Source: Environmental |
| Trichlorofluormethane | 2-Methylheptane | Protection Agency |

The list of organic compounds in the above table plus those that are commonly classified as pesticides are treated as representative of the volatile organic compounds, i.e., VOC's, as hereinafter discussed or referred to.

The problem has recently been reported in the New York Times, Saturday, September 16, 1989, page 52, in an article entitled: "In Battle on Pollutants, Latest Suspect is Rugs." The emission of chemical vapors in closed buildings from newly installed carpeting has proven to be a health hazard as directly experienced by the Federal EPA, at its Washington offices.

The carpet dispute is helping to fuel the growing debate over indoor air quality. Over the last 15 years, many office buildings and houses have been built with an eye toward energy savings. Windows are typically sealed. Ventilation may run only when buildings are occupied.

One result is that millions of office workers breathe 'used' air. Health experts believe that unwanted toxins like cigarette smoke, chemicals and bacteria can build up quickly in such enclosed environments, causing headaches and flulike illnesses.

Though these pollution problems impact on health they also impact on sensitive equipment, such as electronic equipment. For example, *Shields et* al. from Bell Communications Research, Red Band N.J., in a paper entitled "The Effect of Ventilation on the Indoor Concentrations of Vapor Phase Organic Compounds", at page 1, stated the following:

Within telephone switching offices there are many sources of VOC. ---High concentrations of volatile organic compounds are most frequently associated with 'sick building' syndrome... However, they can also pose a threat to the proper operation of modern electronic equipment. They can promote arcing between relay contacts leading to increased contact erosion... Airborne organics can condense on read-write heads,

increasing the likelihood of fine particles sticking to the heads, which can subsequently lead to head crashes and disc drive failures. In short, money saved on energy by reducing ventilation with outdoor air might be lost to increased costs associated with diagnosing and repairing failures of electronic equipment caused by elevated levels of VOC.

The solution to the problem has been one of preventative maintenance, treatment before installation and introducing outside air to the building. All of these procedures are expensive. Many building are so polluted, the cost of refurbishing them is prohibitive. It is very difficult, if not impossible, to legislate to a variety of industries (with a multinational base) to make products used in buildings such that the products do not emit VOC's. The introduction of outside air presumes that the air is sufficiently cleaner than the polluted inside air and circulation will significantly reduce the pollution level. However, introduction of such outside air defeats the energy savings aspect of the building's design and the loss of energy makes this approach economically prohibitive.

Another problem with the dilution of the inside air by the introduction of outside air, which some have urged as a way to control the buildup of VOC's, is the fact that outside air appears to be the source of ozone, another pollutant. According to ***Charles J. Weschler, Datta V. Naik, and Helen C. Shields,*** in a paper entitled: "Indoor Ozone Exposure" soon to be published in JAPCA, ozone contents indoors track the ozone concentrations outdoors, and people are exposed to more ozone pollution by virtue of their longer times spent indoors. According to these authors, "[o]zone is recognized as causing acute and possibly chronic health effects."

Because of this, there is a concern with the use of electronic air cleaners and negative ion generators. According to ***Godish, supra,*** page 280:

Electronic air cleaners and negative ion generators are high voltage devices, and as such are capable of producing ozone. Because of ozone's toxicity at very low concentrations, the use of an air cleaner that itself produces a toxic contamination is of some concern. As a consequence, all devices (to be used indoors) that produce ozone are regulated by the Food and Drug Administration. The FDA ozone limit for electronic air cleaners and other devices is 0.05 ppm; that is, in normal use the device cannot produce ozone emissions that would exceed an indoor air concentration of 0.05 ppm.

It is evident that indoor air pollution is a serious problem that is attracting ever increasing governmental attention (viz., U.S. House of Representatives (100th Congress) bill S.1629), and gives every appearance of not being readily solved or solved by simple means.

A variety of procedures have been used by the art to clean up air. For example, activated carbon has been used for removing VOC's. However, according to ***Ensor et al.,*** in an article entitled "Air Cleaner Technologies for Indoor Air Pollution," Proceeding of the ASHRAE Conference, April 1988, Atlanta, Georgia, published by ASHRAE 1988, Atlanta, Ga., useful activated carbon bed life was found to be short. Breakthrough of such beds has proven to be a problem, causing the authors to indicate that a bed of about 402 m (1/4 mile) in length would be necessary to achieve a 6 month lifetime. Consequently, none of the proposed procedures, including activated carbon, has been considered a solution for sick building syndrome.

This invention is directed to minimizing the indoor air pollution problem by the treatment of circulating air in the building with ozone which, as pointed out by Weschler et al., is itself a significant building pollutant.

The prior art deals considerably with the use of ozone to deodorize air, remove certain volatile organics or reduce biologicals. However, the art is singularly specific about not allowing excess ozone build up in an environment because ozone is a recognized toxicant. Ozone is not allowed to reach a toxic concentration in a room or building when the room or building is being occupied. At some point in time, ozone concentration may reach a danger level and the ozone requires removal. However, at some point prior to that level, some unsuspecting individual could have an adverse reaction to the higher level of ozone present.

According to ***A. Dyas,*** Pathol., 1983, vol. 36, pp. 1102-1104,

Ozone, at high concentration, has been used to disinfect rooms prior to patient occupation,[2] but these concentrations are toxic to man.[7,8] Mortality rates are increased in exercising mice exposed to 0.3 ppm ozone for three hours and then challenging them with an aerosol of ***Strep pyogene.***[9] In mice exposed to 2-5 ppm for five hours and allowed to inhale ***Staph aureus,*** the rate of phagocytosis of pulmonary bacteria slowed compared with controls.[10] In addition Peterson and colleagues[11] studied polymorph function in healthy male human volunteers exposed to 0.35-0.42 ppm ozone for 4 h. They found that, at 72 h. after exposure, both bacterial killing and phagocytosis were moderately but significantly decreased.

The author discussed the useful amount of ozone that one obtains useful bacterial action, and recommended ozone concentrations in the range of between 0.3 to 0.9 ppm, however, the effects of those concentrations on humans were not determined. Moreover, these results were obtained in a smaller space.

This author demonstrates the problems with the use of ozone as a bactericide. It is inherently dangerous to man at high concentrations where it is useful in eliminating biologicals, yet it is apparently dissipated at lower concentrations so that it is ineffective to remove biologicals.

In the hospital room experiments, ozone was undetectable (despite an analyser sensitivity of 0.001 ppm).

Sporadic and inconsistent effects have been reported from the use of ozone to reduce biologicals from air and water. See, e.g.,

*Edelstein et al.,* Appl. Environ. Microbiol., vol. 44, no. 6, pp. 1330-1334, Dec. 1982.

*Ishizaki et al.,* Journal of Applied Bacteriology, 1986, vol. 60, pp. 67-72. The authors in this article provide an interesting discussion on the use of ozone and its comparative effectiveness:

As with other gaseous disinfectants, the sporicidal activity of ozone is affected greatly by humidity. The activity of ozone, as well as that of beta-propiolactone (Hoffman & Warshowsky 1958), increases rapidly with increasing r.h., while there are optimum r.h. ranges in the inactivation of ethylene oxide (Gilbert et al. 1964) and formaldehyde (Anon. 1958; Hoffman & Spiner 1970). The occurrence of considerably long lag phases suggests that the presence of the thick barriers in the surface structure of spores retard ozone penetration to active sites. The presence of a certain amount of moisture in the surface seems to be essential for the rapid penetration of ozone. ----

In addition to increasing the penetrability of ozone, moisture may play a role in the ozone reactions in spore structure. The presence of water often accelerates ozone reactions with organic substances (Hoigne & Bader 1975). Unfortunately, little is known about the sites of ozone reactions which lead to the inactivation of spores. Ozone is highly reactive with many organic substances including biochemical compounds. It reacts with various amino acids in proteins, but sulphur-containing amino acids (cysteine, methionine and cystine) and tryptophane are most susceptible (Mudd et al. 1969; Menzel 1971).

The highest ozone concentration tested in this study was 3.0 mg/l. This level of ozone concentration seems to be attainable when ozone is employed in small rooms or cabinets. Even at such concentration, it is necessary to maintain humidity at more than 80% r.h. At that high r.h., ozone is more active as a sporicide than ethylene oxide, comparable with formaldehyde, and a little less active than beta-propiolactone on the basis of effective concentration levels.

The authors' recommendation of the use of moisture to enhance the effectiveness of ozone as a disinfectant should be contrasted with the call of other authors and researchers to reduce the level of moisture to avoid the generation of biologicals, the very pollutant at issue. Thus one objective of source reduction is the reduction of moisture suggesting that ozone treatment could be ineffective for an overall cure of the problem.

*Elford et al.,* "An Investigation Of The Merits Of Ozone As An Aerial Disinfectant", National Institute for Medical Research, Hampstead, N.W.3, Journal of Hygiene, Vol. 42, No. 3, report the following:

The powerful disinfecting action of ozone has long been recognized, but a search of the literature on the subject reveals considerable differences of opinion as to its value as an aerial disinfectant, when the gas is present in concentrations that could be breathed during prolonged spells with safety. Some authors have been led to dismiss ozone as being quite ineffective, while others have heralded it as having great potential value in ventilation problems. The general problem of aerial disinfection as a practical adjunct to ventilation has many inherent difficulties, and makes demands upon the combined resources of physiological, bacteriological and physico-chemical knowledge. The particular problem of ozone is no exception, and it is evident that failure to appreciate fully its several aspects has led to the conflict of opinion.

Elford et al. inconclusively addressed the issue of what constitutes a safe concentration of ozone.

The physiological properties of ozone, or, more correctly, of ozonized air produced by various forms of ozonizer, have been fairly widely studied. It appears that most people find ozone irritating to the throat when breathed for long spells with the concentration at about 0.05 part per million (p.p.m.) by volume. At higher levels, viz. 0.3-0.5 p.p.m., aggravated symptoms of respiratory irritation and headache develop. In small animals 15-20 p.p.m. brings death within a matter of hours from respiratory congestion (Hill & Flack, 1912). From the recorded evidence, and their own observations, Witheridge & Yaglou (1939) concluded that the safe upper limit of ozone concentration for man is 0.04 p.p.m. This appears to be the only conclusion warranted by experimental work so far adequately recorded.

Claims that higher concentrations of ozone may be safely tolerated have been made, but, unfortunately, they are not supported by detailed scientific evidence.

Elford et al. also addressed the role of humidity on the effectiveness of ozone to biologicals:

The outstanding fact established in these early tests was the dominant role played by humidity in determining the action of ozone, particularly when the gas is present in low concentration. A partial answer to our opening questions was also provided, for it was clearly evident that in relatively dry air, at humidities

less than 45%, ozone, even in concentrations considerably in excess of the tolerance limit, exerts no appreciable disinfecting action on the bacterial aerosols studied. Quite other relationships hold, however, for humidities above 50%, when, in increasing measure as the humidity is raised, the ozone was found to reduce the count of viable bacteria in aerosols sprayed from aqueous, or serum broth suspensions of the organisms. Ozone in concentration less than 0.025 p.p.m. showed definite bactericidal action at 60-80% humidity.

The foregoing experiments have clearly shown that ozone, in concentrations that can be safely tolerated, i.e. not exceeding 0.04 p.p.m., does, in appropriately humid atmospheres, exert a disinfecting action on certain bacteria -- ***Streptococcus salivarius, Streptococcus 'C', Staphylococcus*** albus and ***B. prodigiosus*** -- dispersed in aerosol form as single naked organisms. The disinfecting power of such dilute ozone is still effective against the same organisms, similarly dispersed, but individually coated with a minimal protective covering of protein. As this protective coating increases in thickness however, for instance, with coarse aerosols from suspensions containing appreciable amounts of serum protein, the influence of ozone rapidly diminishes and becomes negligible within the permitted range of concentration.

***Tohru Masaoka et al.,*** "Ozone Decontamination Of Bioclean Rooms," Applied and Environmental Microbiology, March 1982, Vol. 43, No. 3, report

To establish a convenient method for decontaminating bioclean rooms, the effect of ozone at 80 mg/m$^3$ for 72 h was compared with formaldehyde vaporization at an initial concentration of 150 mg/m$^3$ with a gradual decrease to 20 mg/m$^3$ during 72 h. Ozone was found to be inferior to formaldehyde in activity. When the bioclean room was decontaminated twice with ozone, the mean colony count per 10 cm$^2$ was decreased to about the same level as when formaldehyde was used. Ozone had a strong caustic effect upon rubber materials. Despite these disadvantages, ozone decontamination was demonstrated to be superior to formaldehyde vaporization because of convenience, insignificant inhalation of the disinfectant by the hospital staff, and very rapid expulsion of the gas after ventilation. Because the disadvantages of ozone can be easily controlled, this study suggests that ozone decontamination is a promising method for maintaining bioclean rooms.

However, Masaoka et al. noted that the corrosive effects of ozone:

The caustic effect of ozone upon rubber materials was more severe than expected. This indicates the necessity of selecting materials resistant to ozone when bioclean rooms are constructed. The rubber materials in our rooms were replaced with glass wool and vinyl plastics so that the ozone decontamination could be made without any severe effects on the facilities.

As pointed out previously, the U.S. Food and Drug Administration has set an ozone limit for indoor air concentration of 0.05 ppm.

The art describes a number of industrial systems for the treatment of organic pollutants with ozone in the presence of oxidation catalysts, followed by the removal of the ozone with carbon beds. One reference, JP-A-49-84953, Chemical Abstracts, 82(12):76722a, uses ozone in combination with an oxidation catalyst to oxidize carbon monoxide and then the decomposition of the excess ozone with an active carbon. Another reference, JP-A-50-147468, Chemical Abstracts, 84(20):140241p, purifies an exhaust gas containing CO, $NO_x$, $SO_2$, HCHO, $C_2H_4$, etc. by oxidation with ozone in the presence of an oxidation catalyst, and states that the oxidation can be carried out at room temperature. The effluent gas was afterwards passed through an active carbon - silica bed to remove the remaining ozone from the gas. However, the purified gas contained 7-10 ppm of CO, 0.08-0.12 ppm $NO_2$, 3.8-5.1 ppm formaldehyde, 1.3-2.1 ppm methane and 0.02-0.04 ppm ozone. Such an effluent composition fed to an outdoor atmosphere is certainly less onerous than recirculating the composition within an indoor environment. The purified gas stream created by the patentee, as recited in the Chemical Abstracts summery, is the type of air stream that is worse than one seeks to clean up in respect to the sick building syndrome (except that sick building syndrome air streams would not be expected to contain such high concentrations of carbon monoxide). Certainly one would like to not recirculate such a high concentration of pollutants to a facility housing animals and sensitive equipment.

Some have used ozone as a deodorant followed by adsorption of the ozone in an adsorbent. The trouble with such a procedure is that once the adsorbent was saturated with ozone, it has to be replaced. A most popular adsorbent is carbon, especially activated carbon. The problem with activated carbon is that it is susceptible to reaction with ozone, and because the reaction is an exothermic one, the carbon is vulnerable to burning creating a hazardous condition.

A somewhat different approach to deodorization is taken by ***Kanzaki, et at.,*** JP-A-63-126,525, (see Chemical Abstracts, 109, 155422g, 1988) who deodorize air containing industrial toxins ....by passing through an apparatus containing an ozone generator, an oxidation catalyst layer, and an ozone decomposition layer. Thus, a [sic] deodorizing apparatus (44 x 89 x 300 mm) consisted of an ozone generator having plate electrodes and wire electrodes, a $TiO_2 \cdot SiO_2$ catalyst on honeycomb (40 x 85 x 50 mm), and

$TiO_2 \cdot SiO_2$ and MnO ($O_3$ decomposition catalyst) on a honeycomb (40 x 85 x 40 mm). A translation of the Japanese patent application indicates that the length was 20 mm. Air was blown in at 1.7 $m^3$/h and the odor index was decreased from 1000-2000 to 30-50 for 500 h without a decrease in deodorizing power.

The patent application is directed to deodorizing gases containing malodorous constituents and to detoxifying gases containing toxic organic constituents, such as hydrogen sulfide, methyl sulfide, methyl mercaptan, methyl disulfide, dimethylamine, trimethylamine, ethylamine, diethylamine, triethylamine, isobutylamine, pyridine, acetone, methyl ethyl ketone, butyric acid, acetoaldehyde, acrolein, benzene, xylene, toluene, butenes, etc., the typical byproducts found in industrial gas streams. It should be noted that some of these materials are so malodorous in the parts per billion range and their presence in such amounts in an occupied building would be unbearable to the occupants. Moreover, it is well known that hydrogen sulfide is highly toxic, as poisonous as hydrogen cyanide; at concentrations of 10 ppm, it can cause headaches and nausea, at 100 ppm it can be fatal.

The system characterized in this Japanese patent application suggests a number of problems not resolved by this reference:

◊ This Japanese patent application suggests expelling the deodorized air to the outdoor atmosphere because the installations for which the "present invention" of the Japanese patent application are to be employed are not normally those that rely on recycled air. In addition, the Japanese patent application falls to suggest the use of recycled air and, though there is a mention of detoxification of the air, there is no suggestion of any level of purity tied to the deodonzation level achieved. One might contend that the $H_2S$ concentration would be ejected to be reduced below 10 ppm and the other toxins might be expected to have been reduced below their toxicity levels, but there is no data to support those contentions. However, note that with respect to the system of JP-A-50-147468, ***supra,*** the so-called purified air stream contains more pollutants than one would expect to find relative to the issue of the sick building syndrome.

◊ Because the Japanese patent application is directed to deodorization, the catalytic system thereof is limited in what it can do. It is clear the patent application is not addressing the spectrum of VOC's and biologicals of the aerosol type, relating to the sick building syndrome, suggesting that the catalysts are specific to certain malodorous materials. Moreover, deodorization does not mean that the process actually destroys the VOC's that are deodorized. For example, ***Turk et al.,*** "Comparative Odor Control Performance Of Activated Carbon And Permanganated Alumina," Atmospheric Environment, published by Pergamon Press, 1973, vol. 7, pp. 1139-1148, suggests that many deodorizations are the result of chemically converting a compound to another form that has less odor. Visualize the oxidation of formaldehyde to formic acid. This is not the same as removing the pollutants. In effect, deodorization can be a specific dilution process because the molecular concentration of pollutants is not reduced and only the concentration of the species that generates the odor is reduced.

◊ The Japanese patent application uses manganese oxides which have been observed to be ineffective catalysts under typical humid conditions. (JP-A-63-267439 describes a $TiO_2 \cdot SiO_2/\beta\text{-}MnO_2$ catalyst and its use for the decomposition of ozone, especially at temperatures of about 10°C. to about 20°C.).

◊ The class of materials characterized by $TiO_2 \cdot SiO_2$ are highly hygroscopic materials, therefore, these catalyst must pick up a lot of moisture, a condition that suggests that the manganese oxide component must be purposefully dried. (The JP-A just mentioned suggests that in decomposing ozone, such a catalyst may not be so vunerable to moisture.) The added heat would materially add to the cost of such a deodorizing system.

Another system is a room deodorizer to be utilized in unoccupied area (rooms, etc.) sold by A-1 Odor Eliminators, Seattle, WA 98168, in which, during the ozone cycle, a germicidal concentration of ozone is mixed with the air stream and, in the gas phase, the air is deodorized because the "ozone molecules instantaneously catalyze with unseen odor particles." The air stream is then

....re-routed through an isolated chamber. This chamber contains a known vaporous substance that readily reacts with or 'decomposes' ozone molecules. Examples of such substances are water, perfume, or any other oxidizable vapor source. The vaporous airstream is discharged, consuming any residual ozone from the previous cycle.

The patented ozone system developed by A-1 Odor Eliminators generates a short-term, high-strength ozone concentration and then ***immediately*** terminates all residual active ozone in the area. This process allows minute odor, spores, and bacteria particles to be oxidized by ozone ***and*** prevents surface penetration by ozone.

The obvious disadvantages of this system are the facts that -

◊ ozone does not react in a vapor state with may organic materials, including many spores, especially if the ozone is too dry (see *Elsford et al., supra*) and/or the ozone is at a low concentration. Many organic compounds, such as aliphatic hydrocarbons, do not react with ozone in the gas phase.

◊ the elimination of excess ozone is dependent upon the addition of other organic materials which themselves are pollutants. Though one can visualize the use of stoichiometric concentrations of such organic materials, reality dictates that such stoichiometry cannot be obtained or maintained in actual usage.

In summary, ozone is a troublesome toxicant that appears to be an effective biocide at high humidity, must be used in limited concentrations that should be eliminated from the environment before human exposure, and seems not to be superior to other chemicals for such purposes. Some literature has suggested its use with some VOC's, but it has not been shown that ozone is capable of removing VOC's when they are present in low concentrations in an air stream. It is one thing to reduce VOC's in streams at high concentrations, as is the case with treatment of industrial waste streams and it is another thing to remove VOC's from air streams where they are present in low but still undesirable concentrations.

Another problem with the use of ozone for the oxidation of VOC's and biologicals to correct the sick building syndrome, stems from the need to effect any such treatment at low temperatures, such as ambient room or duct temperature, and to effect such treatment with air streams where each pollutant level is in the low or fractional parts per million level on a volume basis. Indeed, the levels of such pollutants are typically less than about 200 parts per billion on a volume basis. An ultimate object is to reduce the amounts of each of the pollutants to below toxicity levels, and certainly it is necessary to reduce the concentrations of the pollutants such that toxin effects are minimized.

Because the volume of air being circulated in such facilities is quite large, one cannot use heat as an aid in the removal of such pollutants without adding materially to the facility's operating costs because the heat added has to be removed so the air supplied to the facility is at the desired use temperature. Prior to this invention, it was thought that without the addition of heat, there is no assurance that the oxidation of the combustible pollutants will be complete, and if it is not complete, then some of the oxidation products will be more onerous pollutants than the materials being oxidized. For example, the low temperature oxidation of hydrocarbons can result in the formation of oxyhydrocarbons, such as carboxylic acids, ketones, aldehydes, and alcohols, to name a few. These are chemicals which may be more difficult to remove than the hydrocarbons from which they are derived and they are more onerous pollutants than the chemicals from which they are derived.

Moreover, the oxidation of biologicals is typically effected in facilities in the absence of the facilities's inhabitants who would be affected by high concentrations of ozone oxidant. Such systems rely wholly on the oxidative effect that gaseous ozone has on the microbes. As pointed out previously, such is dependent upon on the ability of ozone to penetrate the outer layer of the bacteria. However, as pointed out above, ozone requires the presence of moisture to effect significant destruction of such bacteria. It is not established that ozone will effectively attack, e.g., grain negative bacteria (contains lipopolysaccharide-polypeptide complex in the cell wall) so as to remove that endotoxin at such moderate conditions and at a variety of relative humidity conditions.

Important factors in dealing with the sick building syndrome are the variety of pollutants to be dealt with and the necessity of recycling the treated air back into the facility from which it was drawn and subjected to treatment. It is one thing to solve the pollutants issue associated with the sick building syndrome, it is another issue to do so and to also not add to the energy crisis. It is one thing to destroy or modify organic pollutants to deodorize the air, it is another thing to destroy the pollutant to the extent that the air stream can be recycled to a facility that is occupied by humans, other animals, sensitive equipment, foodstuff and the like, without re-introducing another pollutant or higher levels of a different pollutant (than the pollutant being destroyed). It is one thing to solve either the VOC or the biogenics problem, it is another to solve both problems simultaneously.

The literature reflects a current concern over the sick building syndrome phenomena and no single solution is recognized. It is evident from the literature that there are many procedures for treating these pollutants, but none have been applied to solve sick building syndrome. It is also evident from the literature that there is no single procedure for simultaneously eliminating a full range of VOC's and biologicals. Procedures that have been applied to other pollution problems do not seem useful to eliminate the sick building syndrome. Many materials that eliminate pollutants are themselves pollutants; indeed, many are toxicants.

This invention encompasses a process and related apparatus capable of simultaneously eliminating a full range of VOC's and biologicals (viz., bioaerosols, biological products, and the like) within confined areas utilized for societary functions or activities.

The invention relates to a novel method and a novel apparatus for carrying out such method that could solve a major environmental problem which, if left unattended, could become serious enough to have calamitous effects on many people and industries.

This invention relates to solving a long standing and complex problem --- a problem that has defied a solution until this invention. This invention relates to a method which materially mitigates significant aspects of the sick building syndrome. The invention also relates to a process that finds application in general to solving pollution problems associated with large confined areas through which forced air is circulated, and the area is utilized for societary functions or activities, that is, is either inhabited by humans (homes, office buildings, schools, hospitals, factories, and the like) and/or other animals (e.g., livestock, fowl, wildlife, etc. as in the case of zoos, indoor pens, barns, food processing plants, and the like), or is used in the processing of food, pharmaceuticals or involves fermentation processes or contains equipment sensitive to pollutants or contaminants, and the like societary functions or activities. A large confined area is not restricted to any divided space size, and encompasses one large room such as a plant floor or a warehouse space, to an office building or hospital comprised of a plurality of rooms. In each of these situations, the common denominator is their reliance on a common heating and/or cooling system for the area in question and recirculation of air into the area is the method by which the area is heated and/or cooled. The process of the invention treats the circulating air in a catalytic depollution zone removed from contact with animal forms, food, pharmaceuticals, fermenters and/or the sensitive equipment, and the like, that is part of the air circulation scheme of the large confined area facility to remove pollutants and contaminants from the air, and thereafter, the treated air is returned to the cycle flow within the confined area facility located outside of the catalytic depollution zone. As a result, useful activities within the confined area can be carried on while the air serving the confined area is undergoing depollution. In addition, the invention accomplishes this without adding heat of any consequence to the recirculated air stream, and thereby avoids adding significant energy costs to the depollution effort.

More particularly, this invention relates to a process and apparatus that solves volatile organic compound and biological pollution problems associated with large facilities possessing confined areas through which forced air is circulated and in which societary activities are conducted.

Particularly, the present invention provides a process for treating indoor air containing pollutants including volatile organic compounds and biological pollutants comprising the following steps:

(a) withdrawing an indoor air stream from a confined indoor area;

(b) mixing the indoor air stream of (a) with 0.1 to 1500 ppm of ozone;

(c) treating the mixture of step (b) in an air treatment zone containing a solid supported oxidation catalyst which comprises a metal selected from iron, ruthenium, osmium, cobalt, rhodium, iridium, nickel, palladium, platinum, molybdenum and tungsten;

(d) treating the treated mixture of step (c) in an ozone decomposition zone containing an ozone decomposition catalyst selected from nonzeolitic molecular sieves, activated carbon and a supported catalyst comprising a metal selected from nickel, palladium, platinum, manganese, copper and silver so as to obtain a treated air stream containing not more than 0.05 ppm of ozone; and

(e) circulating the treated air stream of step (d) to the confined indoor area of step (a).

The invention recognizes that many pollutants in a closed structure are distributed to the residents, materials and/or sensitive equipment via the circulating forced air stream. The fall run of biogenics and VOC's such as bacteria generated in water deposits, spores introduced from the outdoors, hydrocarbon emissions from automobiles that seep into office buildings, homes, hospitals, factories, ozone created by photochemical action in polluted atmospheres and electric discharge by equipment, formaldehyde released from particle board materials, solvents issuing from carpeting, and the like, are captured in the forced air streams circulating in the typical building and over time, the levels of these pollutants grow to discomforting (or, as for sensitive equipment, deteriorating) levels. The invention involves the treatment of the circulating air stream in a catalytic depollution zone apart from the residents and/or sensitive equipment with ozone at sufficiently high enough concentrations, and in the presence of solid catalysts, to reduce the pollutant levels in the air stream. The same air stream, after ozone treatment, is passed over a catalyst to decompose the ozone and render it impotent. The thus-treated air stream is removed from the catalytic depollution zone and reintroduced to the building structure proper. Though ozone is a toxicant to living matter and a contaminant to sensitive equipment, it may be used to treat the air being circulated in the building in toxifying and/or contaminating amounts for maximum effectiveness in destroying VOC's and biologicals, while at the same time the building structure is being occupied by humans, other animals, foods, pharmaceuticals, fermenter, sensitive equipment, and the like. In addition, though ozone is regarded to be a disinfectant, there are many organic compounds and organic matter that it *per se* will not oxidize sufficiently to combust it to carbon dioxide and water. Thus the overall effectiveness of ozone as a sterilant

or depollutant is dependent in accordance with this invention, upon catalytically activating its oxidizing capabilities.

In addition, the process of this invention finds applications in commercial processes that employ large volumes of sterile air as a reactant or environment for a reaction. Particularly illustrative of such commercial applications are a variety of fermentation processes, viz., microbial expressions of proteins and glycoproteins, foods and beverages, and the like. A special advantage of the invention is the depollution and disinfection of air from a broad spectrum of organic matter without the inclusion of ozone in the air stream that participates as a reactant or mingles with the reaction environment.

This invention relates to a method for purifying the cycled air in a large confined area, as characterized above, which involves treating the air within the confined area to reduce the pollutant levels therein of each of the VOC's to concentrations in the fractional parts per million volume range and simultaneously significantly reduce the biologicals. Also, this invention is directed to a method for maintaining or improving the purity of recycled air in a large confined area, after it has undergone sufficient treatment such that the pollutant level therein of each of the VOC's are at concentrations in the fractional parts per million volume range and simultaneously significantly reduce the biologicals. Furthermore, the invention also encompasses an apparatus for practicing the above method.

The methods for the initial purification of the cycled air and the method for maintaining or improving the purity, as aforesaid, each comprise establishing an independent and confined air replenishment zone within or associated (openly connected) with the large confined area, cycling air containing an amount of pollutants from the large confined area to the air replenishment zone, mixing a controlled amount of ozone with the cycled air within the air replenishment zone, predetermined to be sufficient to reduce the amount of pollutants in the cycled air, passing the mixture of the cycled air and ozone to an oxidation catalyst whereby the levels of the pollutants (exclusive of consideration of the amount of ozone reduced in the cycled air and radioactive materials such as radon which is not affected by the method of this invention. Ozone is typically diminished in concert with the reduction of the other oxidizable pollutants because it reacts with the oxidizable pollutants.) in the cycled air are reduced, passing the cycled air containing residual ozone to an ozone decomposition catalyst under conditions that a predetermined amount of ozone is removed from the cycled air such that the cycled air on return to the confined area, based on the concentration within the total of the confined area, is free of ozone or contains an amount of ozone which is below toxicity levels, and returning the ozone-treated cycled air to the confined area.

The apparatus of the invention comprises a catalytic depollution zone comprising an ozone catalytic treatment zone and a catalytic ozone destruction zone, located in association with the cyclization of air within a large confined structure, wherein the catalytic depollution zone contains:

a) a transport means for the passage of an air stream from the large confined structure to the catalytic depollution zone,

b) a transport means for the pasage of the air stream from the catalytic depollution zone to the large confined structure,

c) an ozone catalytic treatment zone comprising

i) a source of ozone associated with the ozone catalytic treatment zone,

ii) an open connection to the transport means for the passage of air from the large confined structure to the ozone catalytic treatment zone,

iii) a distribution means for mixing ozone with the air introduced to the ozone catalytic treatment zone from the large confined structure,

iv) a solid porous oxidation catalyst layer located downstream of the distribution means and to which is fed the ozone containing air passed from the distribution means, said oxidation catalyst being a supported catalyst comprising a metal selected from Fe, Ru, Os, Co, Rh, Ir, Ni, Pd, Pt, Mo and W,

v) an outlet from the ozone catalytic treatment zone to a catalytic ozone destruction zone,

d) a catalytic ozone destruction zone comprising

i) an open connection to the ozone catalytic treatment zone,

ii) a solid porous ozone decomposition catalyst suitably positioned to make contact with the air stream passed from the ozone catalytic treatment zone to the catalytic ozone destruction zone, said ozone decomposition catalyst being selected from nonzeolitic molecular sieves, activated carbon and a supported catalyst comprising a metal selected from Ni, Pd, Pt, Mn, Cu and Ag,

iii) an outlet from the catalytic ozone destruction zone to the transport means for the passage of the air stream from the catalytic depollution zone to the large confined structure,

e) ozone monitors positioned to determine the concentration of ozone present in the air stream supplied to and removed from the catalytic depollution zone and means for regulation of ozone supplied to and removed from the catalytic depollution zone.

In a preferred aspect of the invention, the catalytic depollution zone apparatus is associated with one or more of the air conditioning and heating systems of the large confined area and it is operated in concert with such systems. In the typical case, the forced air blowers of the furnace or the air conditioning systems will control the air flow rate through the catalytic depollution zone.

In the preferred operation of the process of the invention, the catalytic depollution zone is operated at essentially (or close to) ambient relative humidity, ambient temperature and ambient pressure conditions of the air stream undergoing treatment and this is typically the relative humidity, temperature and pressure of the air stream as it enters or leaves the air conditioning and heating systems with which the catalytic depollution zone is associated and operated in concert. Thus, the catalytic depollution zone may be located on the return air or the forced air side of such furnaces and/or air conditioning equipment serving the large confined area and as part of the air circulation scheme of the large confined area.

In the typical practice of the invention, the catalytic depollution zone of the invention is provided on the return air side of the air conditioning and/or heating systems with which it is associated. As a result, the catalytic depollution zone is operated at a temperature which frequently reflects that within the large confined area. However, there are circumstances where an air conditioning system is located on the roof of the confined area or other exterior surfaces, and in such cases, the catalytic depollution zone may be likewise located in association with the air conditioning zone, or the catalytic depollution zone may be located within the confined area either upstream or downstream of the air conditioning system.

The invention includes the use in the catalytic depollution zone of multiple beds of the various catalysts, including the use of multiple beds in the ozone catalytic treatment zone and the use of multiple beds in the catalytic ozone destruction zone. The multiple beds may be aligned in series (viz., two or more ozone catalytic treatment zones in sequence followed by two or more catalytic ozone destruction zones in sequence), or in an alternating arrangement, such as two catalytic depollution zones aligned in series thereby creating the alternating sequence of ozone catalytic treatment zone and catalytic ozone destruction zone, followed by another ozone catalytic treatment zone and catalytic ozone destruction zone.

The invention includes the use of conventional filtration media or devices located in the apparatus of the invention prior to and/or subsequent to the ozone distribution means in the ozone catalytic treatment zone. In this embodiment the apparatus of the invention comprises:

1) a transport means for the passage of an air stream from the large confined structure to the catalytic depollution zone,

2) a transport means for the passage of the air stream from the catalytic depollution zone to the large confined structure,

3) an ozone catalytic treatment zone comprising

   a) a source of ozone associated with the ozone catalytic treatment zone,

   b) an open connection to the transport means for the passage of air from the large confined structure to the ozone catalytic treatment zone,

   c) a distribution means for mixing ozone with the air introduced to the ozone catalytic treatment zone from the large confined structure,

   d) a filtration means for the removal of particulates from the air stream located before or after the ozone distribution means used to mix ozone with the air, and located prior to the solid porous oxidation catalyst layer located downstream of the distribution means,

   e) a solid porous oxidation catalyst layer as defined above located downstream of the distribution mean and to which is fed the ozone containing air passed from the distribution means,

   f) an outlet from the ozone catalytic treatment zone to a catalytic ozone destruction zone,

4) a catalytic ozone destruction zone comprising

   a) an open connection to the ozone catalytic treatment zone,

   b) a solid porous ozone decomposition catalyst as defined above suitably positioned to make contact with the air stream passed from the ozone catalytic treatment zone to the catalytic ozone destruction zone,

   c) an outlet from the catalytic ozone destruction zone to the transport means for the passage of the air stream from the catalytic depollution zone to the large confined structure,

5) ozone monitors and means for regulation of ozone as defined above.

Figure 1 is a schematic cross-sectional view of a building structure containing a separate confined area and a separate ozone treatment zone for the control and/or reduction of pollutants in the confined area.

Figure 2 is a schematic view of the separate phases of the ozone treatment zone.

Figure 3 is an isometric view of the details of a version of the ozone treatment zone.

Figure 4 is a schematic plan illustration of a laboratory apparatus for carrying out the evaluation of catalysts for use in the invention.

This invention is concerned *inter alia* with the employment of site generated ozone to control the concentration of pollutants of the VOC's and biological varieties in recirculated air with the concentration of ozone below ambient conditions and preferably below toxic limits. The process of the invention divides a facility into a plurality of zones, including a zone involving a confined area in which air is forcedly circulated by one or more fans, which zone is either inhabited by humans (homes, office buildings, hospitals, factories, and the like) and/or other animals (e.g., livestock, fowl, wildlife, etc. as in the case of zoos, indoor pens, barns, food processing plants, and the like), or is used in the processing of food, pharmaceuticals or involves fermentation processes or contains equipment sensitive to pollutants or contaminants (such as computers, semiconductor device manufacture, viz. clean room facilities, and the like). Another zone used in the process is an air treatment zone in which pollutants in at least a portion of the air in the confined area zone are removed from that portion of air by a multi-catalytic treatment comprising catalytic pollutant oxidation with ozone followed by ozone decomposition so that harmful concentrations of ozone are not allowed to build up in the air in the confined zone.

The zone comprising the air treatment step comprises

1. an ozone generator,
2. a mixing sub-zone for mixing ozone and the pollutant containing air,
3. a (porous) oxidation catalyst treatment sub-zone in which the ozone is allowed to oxidize the pollutants such as VOC's and biologicals to carbon dioxide and water, and
4. a (porous) ozone decomposition catalyst sub-zone whereby ozone is decomposed to molecular oxygen.

In the preferred practice of the invention, the step in which the pollutants are oxidized to carbon dioxide and water occurs at moderate temperatures (especially ambient temperatures) such as temperatures of about 10° C. to about 40° C. Also, the preferred temperatures at which ozone is destroyed is about 10° C. to about 40° C. However, both the oxidation and ozone destruction can be carried out under more stringent conditions of temperature, such as temperatures above about 40° C. to about 100° C. through the use of localized heating such that the higher temperatures are maintained at localized hot spots on the surface of the catalyst(s).

*Ozone Generation*

In the typical situation, ozone will be one of the pollutants in the cycled air stream that at some point in time should be removed. Because it is present in the air stream in such an uncertain concentration, one cannot rely on natural ozone pollution to supply all or any predetermined ozone concentration for carrying out the process of the invention. Instead, the predetermined concentration of ozone used in the process of the invention is a combination of that fed via the air stream from the enclosed air environment to be treated, and that generated by any electrical processing in the confined area or prior to the catalytic depollution zone. By monitoring the amount of ozone in the air stream with any one of a variety of ozone detection monitors, the exact amount of ozone present in the stream is easily determined. These monitors can be used to control the amount of ozone that has to be fed to the air stream in order to assure the stoichiometry of the reaction to combust the other pollutants undergoing treatment, viz., the VOC's and the biologicals. Separate from the air stream is provided another source of ozone. It is an ozone generator whose output can be controlled by the ozone monitor that can increase the ozone generation when the ozone levels of the stream are too low, and cut back on ozone generation when the ozone level is too high.

The ozone generation may be effected by any of the well known procedures and equipment for making ozone. The manner of ozone generation is not critical to this invention. For example, ozone may be generated from air or oxygen by the electric discharge process (viz., passage of dry air or oxygen through a high frequency electric field) where the rate and efficiency of ozone production is affected by many variables, such as air flow, percent of oxygen in gas feed, dew point of gas feed, air pressure, cooling water temperature, voltage and frequency applied to electrodes, and ozone concentration. Another technique to produce ozone is the use of ultraviolet lamps over which air is passed. The thus produced ozone may be stored during the operation by adsorption in a bed of granular adsorbent material such as silica gel, as illustrated in US-A-2,872,397, and then later desorbed when needed from the bed by passing air through the bed. Pressure swing adsorption (see US-A-3,663,418 and 4,280,824) may be incorporated into the system for optimum moisture control of the feed of air to the ozone generator and for the production of enriched oxygen streams which can be beneficial to ozone generator performance. It is quite apparent that a small stream of oxygen may be subjected to ozonation to produce a concentrated ozone stream that can be metered into the cycled air stream from the confined area.

Illustrative of commercial ozone generators are those obtained from the following vendors:
Griffin Technics Inc.
Lodi, NJ 07644
Ozone Research & Equipment Corporation
Phoenix, AZ 85019
PCI Ozone Corp.,
West Caldwell, NJ 07006
Polymetrics, Inc
San Jose, CA 95134
The Clean Air Corp.
Lake Forest, IL 60045

*Sub–zone For Mixing Ozone And The Pollutant Containing Air*

In the typical case, air taken from the large confined area that is to be recycled through the heating or the air conditioning system serving the confined structure is passed through a separate filtration step, typically comprising a standard porous filter and/or a standard electrostatic filtration device, in order to remove larger particulates from the air stream. An interesting benefit of the invention results from the ability of the process of the invention to remove ozone from the recycled air. Electrostatic filtration devices typically generate ozone, thus they contribute some of the ozone pollution to the recycled air stream. The ozone removal step of the process of the invention removes such ozone from the air stream. In most cases, such added ozone will be treated as added ozone for use in the ozone catalytic treatment zone and compiled with the excess ozone that is to be removed by the ozone destruction catalyst.

The thus filtered air stream is then mixed with a stream of ozone containing gas. In the preferred operation of this process, the ozone stream is well mixed with the air stream. There are many ways to mix the two streams together in order to satisfy the objective of this process. For example, the two streams can be impinged on solid surfaces and thereby physically intermixed. Illustrative of this procedure is to feed the two streams into rotating fan blades where the blades cause the ozone to become thoroughly blended in the air stream. Another procedure involves feeding the ozone to the interior of a tubular grid chamber which is traversely mounted in the duct carrying the air to or from the furnace and/or air conditioner. The chamber openly-connects the duct on both of its side surfaces because the tubings making up the chamber are spaced apart sufficiently to allow the air stream to move unimpeded through the traversely mounted chamber. The tubing making up the chamber are fitted with small spray nozzles so that the ozone is sprayed uniformly from the interior of the chamber into the air stream. Another device(s) is a spray nozzle located in the center of the air duct from which the ozone is sprayed into the air stream. An alternative is a ring spray device mounted traversely of the air duct with the spray nozzles directed toward the interior of the duct. The spray device may be given a rectangular shape so that the device is mounted flush to the rectangular interior walls of the air duct. The choice of ozone dispersal devices that can be used to blend the ozone with the cycling air stream is almost endless.

The amount of ozone blended with the air stream can be varied considerably. There are a few considerations employed in determining the limits of the ozone concentration introduced to the air stream. They include the efficiencies of the oxidation catalyst and the ozone destruction catalyst that are employed in the system and the amount of ozone monitored after the ozone decomposition step of the process.

For example, highly efficient oxidation catalysts allow the use of less ozone for the oxidation step and this permits the use of less ozone, thereby assuring the preclusion of ozone from the confined area. The less efficient the oxidation catalyst, the more ozone one has to use to effectively remove the pollutants. This imparts the need to more efficiently decompose the additional ozone and thus the efficiency of the ozone decomposition catalyst becomes a more important process variable.

The amount of ozone added to the air stream ranges from 0.1 to 1,500 parts per million volume, preferably 0.2 to 100 parts per million volume, and most preferably, from 0.3 to 25 parts per million volume, and in the most favorable embodiment, from 0.3 to 10 parts per million volume.

A factor that will impact on the amount of ozone that is supplied to the oxidation catalyst discussed below, is the fact that some pollutants react to some degree with ozone in the absence of catalyst, and to some degree some of the pollutant concentration will be lowered simply on the mixing of ozone with the air stream. Obviously, this is a selective reaction carried out in a homogeneous reaction environment. For example, some homogeneous reaction of ozone with benzene, ethylene, methanol, ethanol, and propylene does occur while the reaction of ozone with ethane, propane, butane and acetone requires the use of a catalyst. As the ozone concentration is increased, these VOC concentrations (such as benzene concentration) can be expected to decrease and subject to the amount of ozone that is utilized, the benzene for example may or may not be completely oxidized. However, the concentration of other pollutants such as ethane will remain unchanged as the ozone concentration is varied and acetone reacts homogeneously with the ozone only to a slight extent.

In a practical system, little reliance can be placed on the capability of materially lowering most pollutant concentrations based on the homogeneous reaction thereof with ozone. The flow rates in a practical system would not allow an effective depollution of all of the pollutants including ozone in the time frame of a typical homogeneous reaction system.

*Oxidation Catalyst Treatment Sub−zone In Which Ozone Oxidizes Pollutants*

The ozone laden air stream is fed to the sub-zone containing the (preferably porous) oxidation catalyst. Any solid oxidation catalyst defined above is suitable for the practice of this invention. As a rule, the solid oxidation catalyst is a porous bed of particulate oxidation catalyst or one or more monolithic catalytic structures or coated screens or mesh structures. The solid oxidation catalyst may take any number of shapes, for example, spheres, cylindrical, irregular, platelets, monolithic cylindrical structures, porous screens and meshes, and the like. The density of the bed or other catalyst structure(s) should be sufficient to assure good air passage through the bed or the other type of catalyst structure, and good reaction within the catalyst entity.

The oxidation catalyst is a solid supported catalyst, that is, a solid catalyst deposited on and/or incorporated in and on a solid support. Desirable catalysts comprise the above specified metals in the oxidized or reduced state which are deposited on the support. The oxidation catalyst may be provided on the support in many ways. For example, the oxidation catalyst may be coated onto the support in the conventional manner of making a coated oxidation catalyst. The oxidation catalyst may be included in the support by impregnation to form the conventional structure of an impregnated catalyst.

A particularly preferred oxidation catalyst for use in the invention is a metal oxidation catalyst wherein the metal(s) is one of the aforementioned metals in one or more of its oxide states. The most preferred metal oxidation catalysts are molybdenum, nickel, palladium, platinum, and tungsten in the reduced or oxide forms.

As noted above, the oxidation catalyst may be coated or impregnated on the support. In the preferred practice of the invention, the metal is impregnated on the support using conventional procedures. The choice of the support is not appreciated to be a limiting factor in the operation of the invention. The limited experience with the selection of supports relative to the supports effect on the activity of the oxidation catalyst suggests that the support may range in surface area that is relatively low to an extremely high surface area. In the typical case, the surface area of the support may range from as low as 0.1 square meter per gram (B.E.T. method) and higher. In the preferred case, the catalyst support should have a reasonably high surface area. It is preferred that the metal oxidation catalyst precursor, from a solution, be deposited on an inorganic support that has a relatively high surface area, such as a surface area of at least about 1.5 square meters per gram (B.E.T. method), and more preferably, the surface area is greater than about 2.5 square meters per gram (B.E.T. method). The more active catalysts utilize a support having a surface area of at least about 10 square meters per gram (B.E.T. method), and the most active catalysts utilize a support having a surface area of greater than about 25 square meters per gram (B.E.T. method), indeed more than about 75 square meters per gram (B.E.T. method). The preferred most active catalysts utilize a support having a surface area onto which the solution is deposited, and in contact with, of at least about 100 square meters per gram (B.E.T. method), most preferably at least about 150 square meters per gram (B.E.T. method). Typical support materials suitable for the invention are the silicas, the gamma-aluminas, the titanias, the alumina silicates, the high surface area clays, molecular sieves of the zeolitic and nonzeolitic variety, and the like. Particularly desirable are mixed composite supports in which a high surface area support is deposited over a lower surface area support. For example, a silica gel, alumina silicate and gamma-alumina, and the like, deposited and cured on the surface of an alpha-alumina provides a high surface area for the subsequent deposition of the oxidation catalyst precursor onto the silica gel coating and the thermal stability provided by the low surface area alpha-alumina.

A desirable support material for the oxidation catalyst are the molecular sieves of the zeolitic or nonzeolitic variety. Typical of the molecular sieves of the zeolitic varieties are the chabazite, faujasite, levynite, Linde Type A, gismondine, erionite, sodalite, Linde Type X and Y, analcime, gmelinite, harmotome, levynite, mordenite, epistilbite, heulandite, stilbite, edingtonite, mesolite, natrolite, scolecite, thomsonite, brewsterite, laumontite, phillipsite, the ZSM's (e.g., ZSM-5, (US-A-3,702,886), ZSM-20, (US-A-3,972,983), ZSM-12, (US-A-3,832,449), ZSM-34, (US-A-4,086,186), etc.) and Beta, (US-A-3,308,069 and US-A-28,341

(Reissue)), and the like. Typical of suitable zeolitic molecular sieves employable in the practice of this invention are the following.

A, AgX, AgY, AlHY, alkylammonium X and Y, BaX, BaY, BeY, Ca-A, Ca-germanic near-faujasite, Ca-HX, Ca-X, Ca-Y, calcium $NH_4Y$, CdX, CdY, CeY, CoA, CoX, CoY, CrY, CsL, CsX, CsY, Cu-X, Cu-Y, diethylammonium Y, ethylammonium Y, Fe-X, Fe-Y, HY, KL, KX, KY, L, La-X, La-Y, LiA, LiX, LiY, LZ-10, LZ-20, LZ-105, LZ-210, $MgNH_4Y$, MgHY, MgNaY, $MgNH_4Y$, MgX, MgY, MnX, MnY, Na-A, Na-germanic near faujasite, Na-L, Na-X, Na-Y, $NH_4$-germanic, $NH_4L$, $NH_4X$, $NH_4Y$, activated $NH_4Y$, Ni-A, Ni-X, Ni-Y, omega, PdY, rare earth X, rare earth Y, rare earth ammonium Y, RbX, RhY, SrX, SrY, steam stabilized or ultra-stable Y, tetramethylammonium Y, triethylammonium Y, X Y, Y-82, ZK-5, Zn-mordenite, Zn-X, Zn-Y, the ZSM's, *supra,* and the like.

Other molecular sieves of the non-zeolitic variety include the silica molecular sieves, such as silicalite as depicted in US-A-4,061,724 and 4,073,865. Silicalite has been defined as a "silica polymorph" (see US-A-4,482,774, col. 1, lines 64-68) which has substantially no acid sites. Silicalite's X-ray powder diffraction pattern for the six strongest d-values are:

11.1±0.2Å
10.0±0.2Å
3.85±0.07Å
3.82±0.07Å
3.76±0.05Å
3.72±0.05Å (1Å = 0.1 nm)

The pore diameter of silicalite is about 0.6 nm (6 Å) and pore volume is typically 0.18 ml/g determined by adsorption.

Another silica polymorph related to silicalite is described at column 3, lines 32 to column 4, line 6, of US-A-4,482,774.

Other silica polymorphs, using the definitions ascribed in US-A-4,482,774, include the ZSM's sold by the Mobil Oil Corporation. These may also be used in the practice of the invention in the same manner as silicalite.

New families of crystalline microporous molecular sieve oxides have been recently patented or filed on (by the filing of patent applications), see Table B below, that are based on the presence of aluminophosphate in the framework of the crystal structures. These molecular sieves are actually not zeolites because they are not aluminosilicates and may possess novel crystal structures relative to the known zeolites while others possess framework structures comparable in topology to certain zeolites. (According to *J.V. Smith,* **Amer. Mineral Soc. Spec. Paper** (1963) **1**, 281:
"a zeolite is an aluminosilicate with a framework structure enclosing cavities occupied by large ions and water molecules, both of which have considerable freedom of movement, permitting ion-exchange and reversible dehydration."
See *J. Rabo,* **Zeolite Chemistry and Catalysis**, published by the American Chemical Society, Washington, D.C., ASC Monograph 171, 1979, Chapt. 1, p. 3 (*J.V. Smith*)). For convenience, they are herein characterized as members of the family of "non-zeolitic molecular sieves" which family is generically referred to by the acronym "NZMS". A list of the patents and patent applications covering certain of the NZMSs, and a description of their subject matter, is set out in Table B below.

According to *Flanigen et al.,* in a paper entitled, "Aluminophosphate Molecular Sieves and the Periodic Table", published in the New Developments and Zeolite Science Technology, Proceedings of the 7th International Zeolite Conference, edited by Y. Murakami, A. Iijima and J. W. Ward, pages 103-112 (1986), in respect to the nomenclature of those materials:

The materials are classified into binary (2), ternary (3), quaternary (4), quinary (5), and senary (6) compositions based on the number of elements contained in the cationic framework sites of any given structure. A normalized $TO_2$ formula represents the relative concentration of framework elements in the composition, $(E1_xAl_yP_z)O_2$, where El is the incorporated element and x, y and z are the mole fractions of the respective elements in the composition. Acronyms describing the framework composition are shown in Table A, e.g., SAPO = (Si, Al, P)$O_2$ composition. The structure type is indicated by a integer following the compositional acronym, an integer following the compositional acronym, e.g., SAPO-5 is a (Si, Al, P)$O_2$ composition with the type 5 structure. The numbering of the structure type is arbitrary and bears no relationship to structural numbers used previously in the literature, e.g. ZSM-5, and only identifies structures found in the aluminophosphate-based molecular sieves. The same structure number is used for a common structure type with varying framework composition.

Table A

| Acronyms for Framework Compositions | | | | | |
|---|---|---|---|---|---|
| $TO_2$, T = | Acronym | $TO_2$, T = | Acronym | $TO_2$, T = | Acronym |
| Si,Al,P | SAPO | Me,Al,P,Si | MeAPSO | Other Elements: | |
| | | Fe,Al,P,Si | FAPSO | El,Al,P | ElAPO |
| Me,Al,P | MeAPO | Mg,Al,P,Si | MAPSO | El,Al,P,Si | ElAPSO |
| Fe,Al,P | FAPO | Mn,Al,P,Si | MnAPSO | | |
| Mg,Al,P | MAPO | Co,Al,P,Si | CoAPSO | | |
| Mn,Al,P | MnAPO | Zn,Al,P,Si | ZAPSO | | |
| Co,Al,P | CoAPO | | | | |
| Zn,Al,P | ZAPO | | | | |

In addition, reference may be made to "NZMS-37" or some other numerical value. This refers to that family of non-zeolitic molecular sieves having the 37 structure. The 37 structure correlates in the family of NZMSs those possessing the faujasite structure. Illustrative of these faujasitic materials are the following:

SAPO-37
MeAPSO-37 where Me = Co, Fe, Mg, Mn, Zn
ELAPO-37 where EL = As, Be, B, Cr, Ga, Ge, Li, V, Ti
ELAPSO-37 where EL = As, Be, B, Cr, Ga, Ge, Li, V, Ti

## TABLE B

| Patent or Pat. Applic. No. | Subject Matter of Patent or Patent Application |
|---|---|
| US-A-4,567,029 | MAPO's are crystalline metal aluminophosphates having a three-dimensional microporous framework structure of $MO_2^{-2}$, $AlO_2^-$ and $PO_2^+$ tetrahedral units and having an empirical chemical composition on an anhydrous basis expressed by the formula $mR:(M_xAl_yP_z)O_2$; where R represents at least one organic templating agent present in the intracrystalline pore system; m has a typical value of from 0 to 0.3 and represents the moles of R present per mole of $(M_xAl_yP_z)O_2$; M represents magnesium, manganese, zinc or cobalt, x, y and z represent the mole fractions of M, aluminum and phosphorus, respectively, present as tetrahedral oxides. The fractions are such that they are within a tetragonal compositional area defined by points ABC and D of Figure 1 of the drawings of the patent. |
| US-A-4,440,871 | SAPO molecular sieves are a general class of microporous crystalline silicoaluminophosphates. The pores have a nominal diameter of greater than about 0,3 nm(3 Å). The "essentially empirical composition" is $mR:(Si_xAl_yP_z)O_2$, where R represents at least one organic templating agent present in the intracrystalline pore system; m has a typical value of from 0 to 0.3 and represents the moles of R present per mole of $(Si_xAl_yP_z)O_2$; x,y and z represent the mole fractions of silicon, aluminum and phosphorus, respectively, present as tetrahedral oxides. The fractions are such that they are within a pentagonal compositional area defined by points A, B, C, D and E of the ternary diagram of Figure 1 and preferably within the pentagonal compositional area defined by points a, b, c, d and e of Figure 2, of the drawings of the patent. The SAPO molecular sieves have a characteristic x-ray powder diffraction pattern which contains at least the d-spacings set forth in any one of Tables I, III, V, VII, IX, XI, XIII, XV, XVII, XIX, XXI, XXIII or XXV of the patent. Further, the as-synthesized crystalline silicoaluminophosphates of the patent may be calcined at a temperature sufficiently high to remove at least some of any organic templating agent present in the intracrystalline pore system as a result of such synthesis. The silicoaluminophosphates are generally referred to therein as "SAPO", as a class, or as "SAPO-n" wherein "n" is an integer denoting a particular SAPO as its preparation is reported in the patent. |

| Patent or Pat. Applic. No. | Subject Matter of Patent or Patent Application |
|---|---|
| EP-A-159 624 | ELAPSO molecular sieves have the units $ELO_2^n$, $AlO_2^-$, $PO_2^+$, $SiO_2$ in the framework structure and have an empirical chemical composition on an anhydrous basis expressed by the formula: |

$$mR:(EL_wAl_xP_ySi_z)O_2$$

where "EL" represents at least one element present as a framework oxide unit "$ELO_2^n$" with charge "n" where "n" may be -3, -2, -1, 0 or +1; "R" represents at least one organic templating agent present on the intracrystalline pore system; "m" represents the molar amount of "R" present per mole of $(EL_wAl_xP_ySi_z)O_2$ and has a value from zero to about 0.3; and "w", "x", "y" and "z" represent the mole fractions of $ELO_2^n$, $AlO_2^-$, $PO_2^+$, $SiO_2$, respectively, present as framework oxide units. "EL" is characterized as an element having (a) a mean "T-O" distance in tetrahedral oxide structures between about 0,151 nm (1.51 Å) and about 0,206 nm (2.06 Å), (b) a cation electronegativity between about 125 kcal/g-atom to about 310 kcal/gm-atom and (c) a capability of forming stable M-O-P, M-O-Al or M-O-M bonds in crystalline three dimensional oxide structures having a "m-O" bond dissociation energy greater than about 59 kcal/g-atom at 298K. "w", "x", "y" and "z" represent the mole fractions of "EL", aluminum, phosphorus and silicon, respectively, present as framework oxides.

The "EL" represents at least one element capable of forming a framework tetrahedral oxide and is preferably selected from the group consisting of arsenic, beryllium, boron, chromium, cobalt, gallium, germanium, iron, lithium, magnesium, manganese, titanium and zinc and "w", "x", "y" and "z" represent the mole fractions of "EL", aluminum, phosphorus and silicon, respectively, present as tetrahedral oxides.

| Patent or Pat. Applic. No. | Subject Matter of Patent or Patent Application |
|---|---|
| US-A-4,500,651 | TAPO molecular sieves comprise three-dimensional microporous crystalline framework structures of [$TiO_2$], [$AlO_2$] and [$PO_2$] tetrahedral units which have a unit empirical formula on an anhydrous basis of:<br>$$mR:(Ti_xAl_yP_z)O_2$$<br>wherein "R" represents at least one organic templating agent present in the intracrystalline pore system; "m" represents the moles of "R" present per mole of $(Ti_xAl_yP_z)O_2$ and has a value of from zero to 5.0, the maximum value in each case depending upon the molecular dimensions of the templating agent and the available void volume of the pore system of the particular titanium molecular sieve; "x", "y" and "z" represent the mole fractions of titanium, aluminum and phosphorus, respectively, present as tetrahedral oxides.<br><br>The TAPO molecular sieves are generally further characterized by an intracrystalline adsorption capacity for water at 6.1 mbar (4.6 torr) and about 24°C., of about 3.0 weight percent. The adsorption of water has been observed to be completely reversible while retaining the same essential framework topology in both the hydrated and dehydrated state. |
| EP-A-161 488 | The TiAPSO molecular sieves have three-dimensional microporous framework structures of $TiO_2$, $AlO_2^{\mu008-}$, $PO_2^{\mu008+}$ and $SiO_2$ tetrahedral oxide units having an empirical chemical composition on an anhydrous basis expressed by the formula: |

| Patent or Pat. Applic. No. | Subject Matter of Patent or Patent Application |
|---|---|
| US-A-4,554,143 | $$mR:(Ti_wAl_xP_ySi_z)O_2$$ wherein "R" represents at least one organic templating agent present in the intracrystalline pore system; "m" represents the molar amount of "R" present per mole of $(Ti_wAl_xP_ySi_z)O_2$ and has a value of from zero to about 0.3; and "w", "x", "y" and "z" represent the mole fractions of titanium, aluminum, phosphorus and silicon, respectively, present as tetrahedral oxides and each has a value of at least 0.01. The mole fractions "w", "x", "y" and "z" are generally defined in respect to the ternary diagram of Figure 1 of the applications. |
| | Ferroaluminophosphates (FAPO's) are disclosed in US-A-4,554,143, and having a three-dimensional microporous crystal framework structure of $AlO_2$, $FeO_2$ and $PO_2$ tetrahedral units and having an essential empirical chemical composition, on anhydrous basis, of: $$mR:(Fe_xAl_yP_z)O_2$$ wherein "R" represents at least one organic templating agent present in the intracrystalline pore system; "m" represents the moles of "R" present per mole of $(Fe_xAl_yP_z)O_2$ and has a value of from zero to 0.3, the maximum value in each case depending upon the molecular dimensions of the templating agent and the available void volume of the pore system of the particular ferroaluminophosphate involved; "x", "y" and "z" represent the mole fractions of iron, aluminum and phosphorus, respectively, present as tetrahedral oxides. When synthesized the minimum value of "m" in the formula above is 0.02. The iron of the $FeO_2$ structural units can be in either the ferric or ferrous valence state, depending largely upon the source of the iron in the synthesis gel. Thus, a $FeO_2$ tetrahedron in the structure can have a net charge of either -1 or -2. |
| EP-A-161 491 | The FeAPSO molecular sieves have a three-dimensional microporous crystal framework structure of $FeO_2^{-2}$ (and/or $FeO_2$), $AlO_2$, $PO_2$ and $SiO_2$ tetrahedral oxide units and have a unit empirical formula, on an anhydrous basis, of: |

| Patent or Pat. Applic. No. | Subject Matter of Patent or Patent Application |
|---|---|
| | $$mR:(Fe_wAl_xP_ySi_z)O_2$$ wherein "R" represents at least one organic templating agent present in the intracrystalline pore system; "m" represents the moles of "R" present per mole of $(Fe_wAl_xP_ySi_z)O_2$ and has a value of from zero to about 0.3; the maximum value of "m" in each case depends upon the molecular dimensions of the templating agent and the available void volume of the pore system of the particular molecular sieve involved; and "w", "x", "y" and "z" represent the mole fractions of iron, aluminum, phosphorus and silicon, respectively, present as tetrahedral oxides. |
| EP-A-158 975 | The ZnAPSO molecular sieves comprise framework structures of $ZnO_2^{-2}$, $AlO_2^-$, $PO_2^+$ and $SiO_2$ tetrahedral units having an empirical chemical composition on an anhydrous basis expressed by the formula: $$mR:(Zn_wAl_xP_ySi_z)O_2$$ wherein "R" represents at least one organic templating agent present in the intracrystalline pore system; "m" represents the molar amount of "R" present per mole of $(Zn_wAl_xP_ySi_z)O_2$ and has a value of zero to about 0.3; and "w", "x", "y" and "z" represent the mole fractions of zinc, aluminum, phosphorus and silicon, respectively, present as tetrahedral oxides and each has a value of at least 0.01. |
| EP-A-158 348 | The MgAPSO molecular sieves have three-dimensional microporous framework structures of $MgO_2^{-2}$, $AlO_2^-$, $PO_2^+$ and $SiO_2$ tetrahedral oxide units and have an empirical chemical composition on an anhydrous basis expressed by the formula: |

| Patent or Pat. Applic. No. | Subject Matter of Patent or Patent Application |
|---|---|
| | $mR:(Mg_wAl_xP_ySi_z)O_2$<br>wherein "R" represents at least one organic templating agent present in the intracrystalline pore system; "m" represents the molar amount of "R" present per mole of $(Mg_wAl_xP_ySi_z)O_2$ and has a value from zero to about 0.3; and "w", "x", "y" and "z" represent the mole fractions of magnesium, aluminum, phosphorus and silicon, respectively, present as tetrahedral oxides and each preferably has a value of at least 0.01. |
| EP-A-161 490 | The MnAPSO molecular sieves have a framework structure of $MnO_2^2$, $AlO_2$, $PO_2$, and $SiO_2$ tetrahedral units having an empirical chemical composition on an anhydrous basis expressed by the formula:<br>$mR:(Mn_wAl_xP_ySi_z)O_2$<br>wherein "R" represents at least one organic templating agent present in the intracrystalline pore system; "m" represents the molar amount of "R" present per mole of $(Mn_wAl_xP_ySi_z)O_2$ and has a value of zero to about 0.3; and "w", "x", "y" and "z" represent the mole fractions of manganese, aluminum, phosphorus and silicon, respectively, present as tetrahedral oxides. |

| Patent or Pat. Applic. No. | Subject Matter of Patent or Patent Application |
|---|---|
| EP-A-161 489 | The CoAPSO molecular sieves have three-dimensional microporous framework structures of $CoO_2^{2}$, $AlO_2$, $PO_2$ and $SiO_2$ tetrahedral units and have an empirical chemical composition on an anhydrous basis expressed by the formula: $$mR:(Co_wAl_xP_ySi_z)O_2$$ wherein "R" represents at least one organic templating agent present in the intracrystalline pore system; "m" represents the molar amount of "R" present per mole of $(Co_wAl_xP_ySi_z)O_2$ and has a value of from zero to about 0.3; and "w", "x", "y" and "z" represents the mole fractions of cobalt, aluminum, phosphorus and silicon, respectively, present as tetrahedral oxides, where the mole fractions "w", "x", "y" and "z" are each at least 0.01. |
| EP-A-158 976 | MeAPO molecular sieves are crystalline microporous aluminophosphates in which the substituent metal is one of a mixture of two or more divalent metals of the group magnesium, manganese, zinc and cobalt and are disclosed in US-A-4,567,028. Members of this novel class of compositions have a three-dimensional microporous crystal framework structure of $MO_2^{2-}$, $AlO_2$ and $PO_2$ tetrahedral units and have the essentially empirical chemical composition, on an anhydrous basis, of: |

| Patent or Pat. Applic. No. | Subject Matter of Patent or Patent Application |
|---|---|
| | $$mR:(M_xAl_yP_z)O_2$$ wherein "R" represents at least one organic templating agent present in the intracrystalline pore system; "m" represents the moles of "R" present per mole of $(M_xAl_yP_z)O_2$ and has a value of from zero to 0.3, the maximum value in each case depending upon the molecular dimensions of the templating agent and the available void volume of the pore system of the particular metal aluminophosphate involved; "x", "y" and "z" represent the mole fractions of the metal "M", (i.e., magnesium, manganese, zinc and cobalt), aluminum and phosphorus, respectively, present as tetrahedral oxides. When synthesized the minimum value of "m" in the formula above is 0.02. The as-synthesized compositions are capable of withstanding 350°C. calcination in air for extended periods, i.e., at least 2 hours, without becoming amorphous. |
| EP-A-158976 and EP-A-158348 | "ELAPO" molecular sieves are a class of crystalline molecular sieves in which at least one element capable of forming a three-dimensional microporous framework forms crystal framework structures of AlO2, PO2 and MO2 tetrahedral oxide units wherein "$MO_2$" represents at least one different element (other than Al or P) present as tetrahedral oxide units "$MO_2$" with charge "n" where "n" may be -3, -2, -1, 0 or +1. The members of this novel class of molecular sieve compositions have crystal framework structures of $AlO_2$, $PO_2$ and $MO_2$ tetrahedral units and have an empirical chemical composition on an anhydrous basis expressed by the formula: |

| Patent or Pat. Applic. No. | Subject Matter of Patent or Patent Application |
|---|---|

$$mR:(M_xAl_yP_z)O_2$$

wherein "R" represents at least one organic templating agent present in the intracrystalline pore system; "m" represents the molar amount of "R" present per mole of $(M_xAl_yP_z)O_2$; "M" represents at least one element capable of forming framework tetrahedral oxides; and "x", "y" and "z" represent the mole fractions of "M", aluminum and phosphorus, respectively, present as tetrahedral oxides. "M" is at least one different element ($M_1$) such that the molecular sieves contain at least one framework tetrahedral unit in addition to $AlO_2$ and $PO_2$. "M" is at least one element selected from arsenic, beryllium, boron, chromium, gallium, germanium and lithium, and when "M" denotes two elements the second element may be one of the aforementioned and/or is at least one element selected from cobalt, iron, magnesium, manganese, titanium and zinc.

The ELAPO molecular sieves are generally referred to herein by the acronym "ELAPO" to designate element(s) "M" in a framework of $AlO_2$, $PO_2$ and $MO_2$ tetrahedral oxide units. Actual class members will be identified by replacing the "EL" of the acronym with the elements present as $MO_2$ tetrahedral units.

When "M" denotes two elements "M" may also be at least one element selected from cobalt, iron, magnesium, manganese, titanium and zinc. For example, in each instance "M" includes at least one of the first group of elements, e.g., As, Be, etc., and when two or more elements are present, the second and further elements may be selected from the first group of elements and/or the second group of elements, as above discussed.

The ELAPO molecular sieves have crystalline three-dimensional microporous framework structures of $AlO_2$, $PO_2$ and $MO_2$ tetrahedral units and have an empirical chemical composition on an anhydrous basis expressed by the formula:

| Patent or Pat. Applic. No. | Subject Matter of Patent or Patent Application |
|---|---|
| | $mR:(M_xAl_yP_z)O_2$;<br><br>wherein "R" represents at least one organic templating agent present in the intracrystalline pore system; "m" represents the molar amount of "R" present per mole of $(M_xAl_yP_z)O_2$ and has a value of zero to about 0.3; "M" represents at least one element capable of forming framework tetrahedral oxides where "M" is at least one element selected from arsenic, beryllium, boron, chromium, gallium, germanium and lithium. When "M" includes an additional element such additional elements "M" may be at least one element selected from cobalt, iron, magnesium, manganese, titanium, and zinc.<br><br>The relative amounts of element(s) "M", aluminum and phosphorus are expressed by the empirical chemical formula (anhydrous):<br><br>$mR:(M_xAl_yP_z)O_2$<br><br>where "x", "y" and "z represent the mole fractions of said "M", aluminum and phosphorus. The individual mole fractions of each "M" (or when M denotes two or more elements, $M_1$, $M_2$, $M_3$, etc.) may be represented by "$x_1$", "$x_2$", "$x_3$", etc. wherein "$x_1$", "$x_2$", and "$x_3$", and etc. represent the individual mole fractions of elements $M_1$, $M_2$, $M_3$, and etc. for "M" as above defined. The values of "$x_1$", "$x_2$", "$x_3$", etc. are as defined for "x" hereinafter, where "$x_1$" + "$x_2$" + "$x_3$"... = "x" and where $x_1$, $x_2$, $x_3$, etc. are each at least 0.01.<br><br>The ELAPO molecular sieves have crystalline three-dimensional microporous framework structures of $MO_2$, $AlO_2$ and $PO_2$ tetrahedral units having an empirical chemical composition on an anhydrous basis expressed by the formula:<br><br>$mR:(M_xAl_yP_z)O_2$ |

| Patent or Pat. Applic. No. | Subject Matter of Patent or Patent Application |
|---|---|
| | wherein "R" represents at least one organic templating agent present in the intracrystalline pore system; "m" represents a molar amount of "R" present per mole of $(M_xAl_yP_z)O_2$ and has a value of zero to about 0.3; "M" represents at least one different element (other than Al or P) capable of forming framework tetrahedral oxides, as hereinbefore defined, and "x", "y" and "z" represent the mole fractions of "M", aluminum and phosphorus, respectively, present as tetrahedral oxides. |
| US-A-4,310,440 | $AlPO_4$'s are the basic and simplest of the crystalline aluminophosphate based molecular sieves. They each having a framework structure whose chemical composition expressed in terms of mole ratios of oxides is: $$Al_2O_3:1.0\pm0.2P_2O_5$$ each of said framework structures being microporous in which the pores are uniform and have nominal diameters within the range of about 0,3 to about 1 nm (about 3 to about 10 Å), an intracrystalline adsorption capacity for water at 6.1 mbar (4.6 torr) and 24°C. of at least 3.5 weight percent, the adsorption and desorption of water being completely reversible while retaining the same essential framework topology in both the hydrated and dehydrated state. |
| EP-A-158 350 | SENAPSO are quinary and senary molecular sieves that have framework structures of at least two elements having tetrahedral oxide units "$MO_2^n$" and having $AlO_2^-$, $PO_2^+$ $SiO_2$ tetrahedral oxide units, where "n" is -3, -2, -1, 0 or +1, and have an empirical chemical composition on an anhydrous basis expressed by the formula: |

| Patent or Pat. Applic. No. | Subject Matter of Patent or Patent Application |
|---|---|
| | $mR:(M_wAl_xP_ySi_z)O_2$<br>wherein "R" represents at least one organic templating agent present in the intracrystalline pore system; "m" represents the molar amount of "R" present per mole of $(M_wAl_xP_ySi_z)O_2$ and has a value of from 0 to about 0.3; "M" represents at least two elements selected from arsenic, beryllium, boron, chromium, cobalt, gallium, germanium, iron, lithium, magnesium, manganese, titanium, vanadium, and zinc; "n" is as above defined; and "w", "x", "y" and "z" represent the mole fractions of elements "M", aluminum, phosphorus and silicon, respectively, present as tetrahedral oxides, each having a value of at least 0,01. |

*Oxidation Catalyst Preparation*

The oxidation catalyst is typically prepared from hydrolyzable or condensable forms of the various metals used as catalysts. In the typical case, the hydrolyzable form comprises

$MX_{a/b}$

wherein M is the metal atom possessing a charge such as a valence charge or hydrogen bonding charge or van der Waal forces charge, and the like which has the value of *a*, X is the hydrolyzable or condensable component, and *b* is a number representing the complimentary countercharge of X. X may be one or more of a variety of hydrolyzable groups, such as halogen (viz., chlorine, iodine, bromine, or fluorine), nitrate, sulfate, oxalate, ammonium, propionate, maleate, phosphate, acetylacetonate (in which case the metal is complexed to the acetylacetonate), and the like. Hydroxyl is illustrative of a suitable condensable group. In the preferred aspect of the invention, the metal nitrate salts of various metal ions are used in supplying the metal to the support.

The hydrolyzable or condensable form of the metal is typically dissolved in a solvent for the compound and that solution is used to impregnate the support. Suitable solvents comprise a wide range of polar materials such as water; ketones such as acetone, methyl ethyl ketone; alcohols such as methanol, ethanol, ethylene glycol, polyethylene glycol; acids such as acetic acid, propionic acid; amines such as ethanol amines, e.g., monoethanolamine, diethanolamine, triethanolamine, alkylene amines, such as ethylene diamine, diethylene triamine, triethylene tetramine, and the like.

The amount of catalyst provided in the oxidation catalyst is not limited to any specific amount. Certainly, the effectiveness of the oxidation catalyst is tied to the specific metal and the amount deposited onto and into the support, and the amount of supported catalyst used in the process of the invention to the total amount of pollutants being oxidized. Thus, the manner in which the process is practiced in any specific case will dictate to a large degree the choice and amount of catalyst.

However, in general, the amount of catalyst provided in the supported oxidation catalyst may vary widely, depending on those factors mentioned above. For example, the amount of the catalyst (whether there is used one or more of the catalysts in the supported oxidation catalyst) may range, based on the weight of the metal making up the catalyst, from about 0.01 to about 70 weight percent of weight of the supported oxidation catalyst. The concentration of the catalyst in the depollution zone is dependent upon the amount of catalyst in the supported oxidation catalyst and the amount of the latter provided in contact with the air stream cycled through the depollution zone. In the more typical case, the amount of the catalyst provided in the supported oxidation catalyst may range from about 0.02 weight percent to about 60 weight percent, based on the weight of the supported oxidation catalyst. In the more preferred cases, the amount

of the total catalyst provided in the supported oxidation catalyst ranges from about 0.1 to about 50 weight percent, based on the weight of the supported oxidation catalyst.

It should be appreciated that no one catalytic species may be optimum to oxidize all of the pollutants to $H_2O$ and $CO_2$. This invention may be practiced, in a preferred manner, by the use of catalysts that contain a number of catalytic species. It has been found that such a combination is more useful than each catalytic species when used alone.

The impregnation step simply involves covering or coating the support with the solution of the oxidation catalyst precursor and then drying the coating on the support to fix it. The drying temperature is not narrowly critical and can range from about 50 °C. to about 150 °C. for a period of about 1 hour to about 48 hours. The lower the temperature, the longer will be the heating period and the higher the temperature, the shorter will be the heating period. The drying step may be carried out in stages, such as a first stage to remove water and a second stage to fix the catalyst. Thereafter the dried catalyst is subjected to calcination or roasting through heating at an elevated temperature such as from about 200 °C. to about 900 °C. for about 1 minute to about 16 hours, with lower temperatures taking longer heating periods and higher temperatures taking shorter heating periods. Heating may be carried out in the presence or absence of air. If it is desired that the catalyst be in a reduced form, then heating should be carried out in the absence of oxygen such as in an inert atmosphere or in a hydrogen atmosphere. However, if the catalyst is to be oxidized or it is irrelevant because heating is effected at a high enough temperature to preclude oxide formation other than some possible surface oxide formation (viz., as in the deposition of silver), then of course, heating can be effected in an oxygen containing atmosphere.

The following is illustrative of a useful procedure for making a supported oxidation catalyst:
A known amount of the desired metal salts, such as the metal nitrates, is dissolved in a solvent, typically water. The amount of solvent should correspond to the pore volume for the support used. The support is placed in a breaker which is subsequently evacuated if the support requires this technique for impregnation. The metal ions dissolved in the solvent are then injected onto the support. The impregnated support is dried at 100 °C overnight and calcined overnight at 400 °C. If, due to the desired loading and to the solubility restrictions of the system the resultant volume of the metal ions and the solvent is greater than the pore volume of the support, only that portion of the solution which will be adsorbed by the support is injected onto the support. After drying at 100 °C overnight, the impregnation and drying steps are repeated until all of the solution is adsorbed by the support before the calcination step. In the preferred procedure, the various components that are impregnated into the support are coincidentally (i.e., simultaneously) deposited into the support and thereafter the impregnated support is dried and calcined (roasted). An alternative procedure which essentially achieves the same results, is to sequentially deposit soluble salts or complexes of the metal into the support, and after each deposition, the support is dried at a low temperature to remove solvent, then the support is reimpregnated with the same or another catalyst salt or complex in the same solvent followed by more drying, and ultimately by calcining at the elevated temperatures. This sequential deposition results in the resolubilization of the initially deposited salt or complex in the solvent containing the other aliquot of salt or complex, and the combination is coincidentally deposited into the support. The last technique involves post addition in which metal salt or complex is impregnated as aforesaid and the treated support is dried and calcined, and then the calcined support is again treated with metal salt or complex and the resultant impregnated catalyst is dried and calcined. The post addition catalysts are typically not as active as the other types describe above.

*The Ozone Decomposition Catalyst*

The filtered and oxidation catalyst treated air stream is passed to an ozone destruction zone in which there is provided an ozone decomposition catalyst. The air stream contains the residual ozone well mixed.

The amount of ozone in the air stream can vary considerably. There are a few considerations employed in determining the amount of ozone concentration in the air stream. They include the efficiencies of the oxidation catalyst, the amount of ozone added to the air stream before the oxidation catalyst treatment, the concentration of pollutants in the air stream, and the ambient concentration of ozone in the air stream prior to treatment with the oxidation catalyst.

In the typical case, the amount of ozone in the air stream after oxidation catalyst treatment ranges from about 0.01 to about 500 parts per million volume; preferably, the amount of ozone in the treated air stream ranges from about 0.02 to about 25 parts per million volume; and most preferably, the amount of ozone in the air stream ranges from about 0.02 to about 10 parts per million volume.

The ozone containing air stream is fed from the sub-zone containing the oxidation catalyst to the sub-zone containing the ozone decomposition catalyst. Any of the above specified solid ozone decomposition

catalysts is suitable for the practice of this invention. As a rule, the solid ozone decomposition catalyst is a particulate form of the material that is deposited on particulate or nonparticulate support material or is unsupported. Generally, the solid ozone destruction catalyst is a particulate form of the catalyst deposited on support materials to form either a porous bed or a monolithic structure. The solid ozone destruction catalyst may take any number of shapes, for example, spheres, cylindrical, irregular, platelets, and the like. The density of the bed should be sufficient to assure good air passage through the bed and good reaction within the bed.

The ozone decomposition catalyst is desirably a solid supported catalyst, that is, a solid catalyst deposited on and/or incorporated in a solid support, or it can be the support *per se* that contains as a structural component the catalyst in a form which allows it to destroy ozone under the conditions employed. In one embodiment of the invention, desirable catalysts comprise certain metals in the surface oxidized or reduced state that generates a base or acid condition which are deposited on the support. The ozone decomposition catalyst may be provided on the support in many ways. For example, the catalyst may be coated onto the support in the conventional manner of making a coated catalyst. The catalyst may be included in the support by impregnation to form the conventional structure of an impregnated catalyst.

There is substantial art concerning methods for the destruction of ozone into molecular oxygen, via the reaction:

$$2O_3 \rightarrow 3O_2 + \text{energy}$$

Overall, techniques employed by the prior art may be employed in the practice of this invention to destroy excess ozone after the catalytic oxidation step of the pollutants. However, it is desired to effect ozone destruction with a minimum of energy input. In particular, it is desired that the ozone destruction be effected in an ozone destruction zone that operates at about ambient temperature, which in the usual case is close or at room temperature (viz., 23-25 °C.) on the return air side and higher or lower on the forced air side. In addition, the zone could be used in exterior (e.g., outdoor) sites and therefore, could be employed at temperatures ranging from about 10 °C. to about 40 °C. Higher temperatures are contemplated when the activity of the ozone decomposition catalyst is insufficient to remove enough of the ozone below any toxicity level. Increasing the temperature of the catalytic ozone destruction zone will inevitably increase the activity of the ozone decomposition catalysts. It is not always necessary to increase the temperature of the catalytic ozone destruction zone in order to improve the activity of the catalyst. For instance, the catalyst can be subjected to magnetic radiation such as infrared radiation, ultraviolet, X-ray (see JP-A-51-010174) and/or microwave radiation, to enhance the ozone destruction capabilities of the catalyst. Magnetic radiation can result in the heating of the catalyst; however, the level of heating of the catalyst zone can be carefully controlled to a minimum level, i.e., a sufficient amount of heat to assure the desired degree of ozone destruction.

The ozone decomposition catalyst need not be a metal material such as employed for the oxidation catalysts discussed above. For example, certain support materials are suitable ozone destruction catalysts because they have the capacity of adsorbing ozone in cavities where the ozone becomes trapped and therein undergoes destruction because of the presence of a catalytic species within the structure of the cavities. Particularly desirable ozone decomposition catalysts are the nonzeolitic molecular sieves (NZMS), especially those of the small or medium pore varieties, especially the small pore NZMS's. Especially preferred are chabasitic and erionitic materials of the NZMS types, such as SAPO-34, CoAPSO-34, MnAPSO-34, SAPO-17, ALPO-34, ALPO-17, ALPO-11, ZnAPO-11, SAPO-11, and the like. Also desirable are the medium and large pore molecular sieves such as ZSM-5, and molecular sieves such as LZ-10, LZ-20 and LZ-210, and the like. The pores of these materials are large enough to allow the ozone to penetrate into them. When the sieve is acidic, it possesses the capacity of decomposing ozone more readily by causing the ozone to react to generate diatomic oxygen. According to JP-A-52-81063 water added to the zeolite bed enhances ozone decomposition.

Particularly preferred as ozone decomposition catalysts are the above metal catalysts where the surface metal atoms are in a form that is capable of accepting more oxygen such as chemisorbed oxygen or provides a reaction site for the ozone, with the net effect being the conversion of the ozone into dioxygen (diatomic oxygen). Important to the function of such catalysts is the receptivity of the metal surface to accept the triatomic ozone and discharge it as molecular oxygen (dioxygen). Such metals are typically of the bulk metal variety, that is, the metal has sufficient metal to metal bonds to be classed a bulk metal compound.

A particularly desirable ozone decomposition catalyst is a silver metal containing catalyst, particularly a silver metal supported catalyst where the support has a surface area ranging from about 0.1 to about 350

$m^2/g$, preferably in excess of 4 $m^2/g$ though good results have been obtained with a silver metal catalyst deposited on an $\alpha$-alumina support having a surface area in the range of about 2-4 $m^2/g$. Most suitable silver catalysts are metal deposited in bulk form on a porous support, preferably having a high surface area such as silicas, gamma-aluminas, aluminum silicates (aluminosilicates), titanias, zirconias and the like. It is frequently desirable to use a silver containing catalyst that contains a promoter or modifier, such as alkali metal and mineral acid cations. Silver catalysts are particularly desirable because they act as a sponge for atmospheric halogen such as chlorine and bromine, and halides are active reactants with ozone to convert it to molecular oxygen. Such catalysts typically contain from about 0.1 to about 50 weight percent of silver based on the weight of the total supported catalyst. Preferred versions of such catalysts typically contain from about 0.2 to about 20 weight percent of silver based on the weight of the total supported catalyst. More preferred versions of such catalysts typically contain from about 0.2 to about 12 weight percent of silver based on the weight of the total supported catalyst. Most preferred versions of such catalysts typically contain from about 0.5 to about 8 weight percent of silver based on the weight of the total supported catalyst. The preferred silver containing catalysts are supported silver catalysts in which silver is impregnated into and onto the surfaces of the support, and the support is a high surface area support. The high surface area supports provide greater reactivity at lower temperatures of the catalyst for the ozone than do the lower surface area supported silver catalysts though the lower surface area supports are effective and are encompassed by the invention. As especially efficacious supported Ag catalyst is silver deposited on NZMS's where the support adsorbs the ozone and the silver serves to destroy the ozone.

Illustrative of a particularly useful catalyst is a simple catalyst formed by the deposition of platinum black on glass wool support. This catalyst has proven to be most effective in reducing ppm by volume concentrations of ozone to ppb by volume concentrations, indeed below the detection limits of the analytical technique employed.

It is believed that these metal catalysts provide surfaces that adsorb oxygen (derived from ozone) yet the surface is not in a fully covalently oxidized state. This means the surface metal atoms are capable of either chemically decomposing ozone in favor of monooxygen that becomes weakly bonded to one or more of the metal atoms at the surface eventually to be pumped out as diatomic oxygen or the surface metal atoms are complexed with acidic or basic components loosely held on the metal surface and they react with the ozone to decompose it to water and diatomic oxygen.

Ozone is readily adsorbed by silicas, aluminosilicates and zeolitic molecular sieves. It is also known to combine molecular sieves with carbon, particularly activated carbon, for use in destroying ozone. In this regard, reference is made to the following patents:

US-A-2,872,397
US-A-2,980,494
US-A-3,006,153
US-A-3,134,656
US-A-3,663,418
US-A-4,101,296
US-A-4,259,299
US-A-4,280,824
US-A-4,619,821
DE-A-26 10 227
JP-A-53-130270
JP-A-50-147468

There are many references that suggest the use of activated carbon to destroy ozone. In this case, the carbon may become a reactant and the process of the reaction is conventional combustion, and as with any combustion, if the combustion gets too exothermic, then the carbon bed can be engulfed by flames, serving to burn up the activated carbon bed and inflicting substantial damage to the surrounding environment. However, activated carbon, if properly employed can be an effective, ***albeit*** less desirable than other systems, for the control of ozone. In this respect, see the following:

JP-A-50-147468

JP-A-58-043234, describes an ozone destruction catalyst obtained by adding 1-30 atom % NiO (as Ni) and 0.1-20 wt % Ag to cobalt oxide. Illustrative of how the catalyst can be made is the following:

Calcine basic cobalt carbonate ($CoCO_3 \cdot Co(OH)_2$) at 330 ° C. in air for 48 hours to obtain $CoO_x$. Calaine NiO(OH) at 200 ° C. in air for 3 hours to obtain NiO. Mix 5 atoms of NiO (as Ni) with $CoO_x$ to obtain a mixture and 20 wt. % silica sol is mixed with the mixture, and the combination is calcined at 250 ° C. in air for 3 hours, and divided to obtain a powdery $CoO_x$-NiO catalyst of 10-12 mesh. The catalyst is immersed in aqueous solution of silver nitrate, dried at 150 ° C. for 3 hours, and calcined at 250 ° C. in air for 6 hours to

obtain a Ag-$CoO_xO$ catalyst.

JP-A-85-050492 (also see JP-A-57-130650) describes a "catalyst for decomposing ozone exhausted from waste gas treatment" containing a mixed deposit of Pt and Re on a catalyst carrier in the atomic ratio of Pt:Re of 2:1-10:1. An example of how the catalyst is made is the following:

Mix active carbon powder of below 5 microns particle size with a little ion exchanged water, divided in a ball mill and mixed with more ion exchanged water to obtain and aqueous slurry of active carbon and silica. Immerse a glass fiber net calcined at 300-500 °C. in air in the aqueous slurry and dry to obtain an active carbon deposited glass fiber net. Immerse the resulting net in a hydrochloric acid solution containing rhenium chloride and chloroplatinic acid, and calcine at 300 °C. for 5 hours to produce a Pt and Re deposited net type catalyst.

Ozone may be decomposed by reaction over the oxide type supported metal catalysts such as the oxide type supported metal oxidation catalysts described above. The problem with the use of such catalysts for the destruction of ozone is that they typically require supplying substantial amounts of heat to the ozone destruction zone, see e. g., JP-A-51-004094. The use of active manganese dioxide to destruct ozone apparently requires temperatures in the range of 120-150 °C.

There are a number of publications directed to portable air filtration devices such as one sold under the name Instapure™ which cite the use of, e.g., a 50:50 mixture of activated carbon with a low-temperature catalyst consisting of copper and palladium salts deposited on porous alumina. It is alleged to remove carbon monoxide, ozone, sulfur dioxide, hydrogen sulfide, ammonia and benzene at an air flow rate of 8.1 $m^3$/min. (300 $ft^3$/min.). Hazardous components of tobacco smoke were alleged to be effectively reduced using a small ancillary-filter containing the low-temperature catalyst (see *Collins,* Chemical Abstracts, **107**-(24):222452r). An $Al_2O_3$-supported $CuCl_2$-$PdCl_2$ catalyst for portable air cleaners is stated to remove CO by oxidation at 25-400 °C., $NO_2$ and $SO_2$ by adsorption until saturation, and $H_2S$ by reaction to form CuS. HCN, methane, isobutane, and *n*-$C_3H_8$ are reported to be also oxidized at elevated temperatures (see *Collins,* Chemical Abstracts, **105**(8):65647x).

JP-A-62-266243 (see Chemical Abstracts, **108**(26):227540y) discusses an oxidation catalyst from transition metals and/or their oxides that removes CO by oxidation to $CO_2$ and $O_3$ by decomposition. The catalyst is a Cu-Mn system oxidation catalyst provided in a honeycomb form in a support material made of $SiO_2$, $Al_2O_3$ and MgO. It is alleged to destroy 100% of the ozone over a period of 100 hours of testing.

JP-A-63-12347 describes (as reported by Chemical Abstracts, **108**(22):192035h) a catalyst for decomposing ozone in the following terms:

The --- catalyst is composed of an active catalyst substance comprising ≧ 1 element selected from Pt, Pd and Rh, $CeO_2$, and an active $Al_2O_3$ loaded on a heat-resistant three-dimensional catalyst support. The catalyst is active at a low temperature of 20-100 °, and durable. Thus, an activated $Al_2O_3$ was impregnated with a solution containing $Pd(NO_3)_2$, $Rh(NO_3)_2$ and $Ce(NO_3)_2$, dried and baked at 600 °, then milled in a dilute $HNO_3$ to make a slurry. A cordierite honeycomb support (93 mm outer diam x 102 mm long cylinder having 300 cells/$in^2$) was dipped in the slurry, blown with compressed air, and dried at 130 ° to give a catalyst loading $Al_2O_3$ 73, $CeO_2$ 7, Pd 0.9 and Rh 0.1 g/L. $O_3$ decomposition tests <$O_3$ 200 ppm, 0.8 m/s (SV = 57,000 $h^{-1}$), 20-100 °〉 with the catalyst (50 mm long) showed $O_3$ decomposition of 90, 97 and 99% at 20, 50 and 100 °, respectively vs. 74, 82 and 91% for a control prepared in the same method as above but without $Ce(NO_3)_3$.

US-A-4,184,983 describes the use of a composited carbonaceous component with a metal from Group VIII of the Periodic Table to remove ozone from a gas stream.

There are a number of commercial catalyst that are proposed for use in the decomposition of ozone. Carulite®, a manganese dioxide-based catalyst, sold by the Carus Chemical Company, LaSalle, IL 61301, is an industrial type ozone decomposition catalyst that appears to be effective at elevated temperatures. It is also recommended for oxidation of a variety of VOC's at elevated temperatures. See US-A-4,290,923 and 4,299,735. Mentioned in the patents is the Hopcalite family of catalysts, another of the same type of oxidation catalyst.

An alternative to the use of ozone decomposition catalyst is to utilize a split feed of incoming air to the oxidation catalyst wherein a portion of the cycled air is oxidized with ozone in the presence of an oxidation catalyst and the remaining portion of the cycled air stream is caused to bypass the oxidation catalyst in this instance. Subsequent to the oxidation catalyst zone may be provided an ozone monitor and if the ozone content in the stream subsequent to the ozone oxidation zone exceeds the toxicity level for recycle into the living or work area then the monitor causes the diluted stream of the effluent from the oxidation catalyst zone plus the bypass air stream to be recycled to the oxidation zone so that the pollutants in the air stream now being fed to the oxidation zone exceeds the stoichiometry of the ozone concentration. Consequently, the ozone level is reduced in the oxidation step. This procedure presumes that the oxidation and

decomposition step will not effect total elimination of the pollutant level on each pass and that over a period of time, the pollutant level will be reduced to safe levels and maintained as such thereafter unless the pollution level is dramatically increased. In this case, the recycle of the air stream to the oxidation catalyst zone is the equivalent of using at least two catalysts for the reduction of the pollutants and ozone.

*Apparatus*

As discussed above, the invention treats air taken from a confined area and cycles it to an air replenishment zone where the air is treated with ozone in the presence of an oxidation catalyst followed by treatment by an ozone decomposition catalyst. The resultant air is replenished and returned to the confined area. In the lexicon of this invention, the confined area is a large space(s) that is heated or cooled by forced air from a separate location that is independent of the confined area. For example, an office building that uses forced air heating or cooling to heat or cool the multi-roomed building would represent a confined area according to the invention. A large warehouse space or a large operating room or a large clean room, each of which house sensitive equipment, or is used by or for animals and/or the handling or storage of food, can be a confined area according to this invention. On the whole, a confined area is a reasonably large space or spaces represented by a volume typically of at least about 270 $m^3$ (10,000 $feet^3$), and may contain more than one room. The typical confined area contains more than one room that is supplied heated or cooled air from a central source located within the confined area or associated with the confined area.

The air replenishment zone may reside within the confined area and, from a practical standpoint, always resides as part of the air recycle between the confined area and the heater(s) and/or air conditioner(s) used in the heating or cooling of the confined area. The air replenishment zone need not be incorporated in the incoming or outgoing duct work of a furnace or air conditioner. For example, the air replenishment zone may be located outside of such duct work and receive a slip stream of air from the duct work that is rendered more pure in the air replenishment zone and thereafter returned to the duct work.

The air replenishment zone is typically a passageway through which the cycled air from the confined area is paced on its way to or from the furnace(s) or air-conditioners serving the confined area. That passageway may be divided into an pollutant oxidation zone, where the pollutant oxidation catalyst is located, and the ozone destruction zone, where the ozone decomposition catalyst is located. The passageway is served by at least one ozone generator which is provided such that ozone that is generated is well distributed within the cycled air stream upstream of the pollutant oxidation zone. As a result, the ozone is mixed with the cycled air prior to the pollutant oxidation zone and the mixture is fed to the pollutant oxidation zone.

As pointed out above, the oxidation catalyst and the ozone destruction catalyst are typically supported materials which provide a substantial surface area to the air stream passing through it. If the supported material has a substantial surface area, some of which constitute pores in which the air stream passes, then the air stream makes maximum contact with the surface and effects a substantial residence time within the beds by virtue of the circuity of passage through the bed.

Figure 1 shows a schematic cross-sectional view of a building structure 1 defining a confined area and containing a separate ozone treatment zone 3 for the control and/or reduction of pollutants in the confined area. In this illustration, the ozone treatment zone 3 is attached to the furnace/air conditioner 5 in the basement portion of building 1. Building 1 could be an office building or warehouse or residential structure. Duct passages 7 symbolically characterize forced air transmitted throughout the building with return air ducts 9 symbolically characterizing the return of air to the ozone treatment zone 3 and thence to furnace/air conditioner 5. Obviously, the ozone treatment zone could be as well located on the forced air side of furnace/air conditioner 5 or it could be located in both the return air and forced air sides.

Figure 2 is a schematic perspective view of the separate phases of an ozone treatment zone. In the main, this ozone treatment zone is located in metal duct 11 which may be a return air or forced air plenum chamber within duct work leading to or leading from the furnace or air conditioner system of the confined area, such as the building structure of Figure 1. Air passage, in the direction of the arrows, from the interior of the building or from the furnace/air conditioner, in the air cycle within the building, is carried through open area 10 of duct 11 to a porous filtering stage 13. This filter can comprise any of the mechanical type filters such a fiber glass mat filter, or an electrostatic filtration device. To the extent the air stream contains ozone or ozone is added by the action of an electrostatic filtration device, such is taken into account in determining the amount of ozone to be subsequently added to the air stream. The purpose of the filtration is to remove larger airborne particulate matter such as fibrous materials, dust, etc. from the air stream. As pointed out previously, the filtering stage may be position after the air stream and the ozone are mixed, which in this case would occur after the ozone diffuse 15, *infra*. The partially cleansed air stream is then

passed across ozone monitor 33 of a conventional commercial design that ascertains the ozone concentration in the stream, a determination used for the adjustment of the amount of ozone to be added subsequently to the air stream. Then the air stream is passed to an ozone diffuser 15 containing a plurality of nozzle tubes 23 containing spray devices through which ozonized air is fed in an uniform manner to the air stream. The ozonized air is formed in ozone generator 21 outside of duct 11 and fed via pipe 19 to manifold 17 through which the ozonized air is essentially uniformly distributed within the open interior of diffuser chamber 15 thence to the nozzles 23.

The operation of diffuser 15 is more particularly set forth in Figure 3. Figure 3 represents a facing view of a typical ozone diffusion device by which ozone can be uniformly dispersed in the cycled air stream in a manner whereby to uniformly distribute ozone in the air stream and thereby effect the most uniform treatment of the air stream. As shown in Figure 3, ozonized air is fed from the ozone generator 21 (see Figure 2) through pipe inlet 19 into enclosed manifold 17. Manifold 17 is a walled-in chamber and contains a plurality of baffle plates 24 aligned so as to deflect the ozonized air within the chamber to effect a uniform upwardly distribution of the ozonized air toward porous diffusion zone 26. Porous diffusion zone 26 comprises a layer of macroreticulated ozone resistant foam or an open pore metal fritted diffusion plug that introduces a small pressure drop on the ozonized air flow toward diffuser 15. The layer of macroreticulated foam or plug is open-celled and consequently provides passage of the ozonized air therethrough. Openly connected to manifold chamber 17 are a series of hollow tubes 23, typically made of metal or plastic, parallelly aligned in area 15. They, the hollow tubes 23 and the space between the tubes 23, comprise diffuser 15. Each hollow tube 23 is provided with small nozzle arrangements. The nozzles may be simply holes in the hollow tubes or conventional spray nozzle structures. In the typical design, the holes in the tubes are made progressively larger as the holes become more distant from manifold 17. This allows for more uniform dispersal of the ozonized air into the interior spaces of diffuser 15. As the cycled air stream is passed through diffuser 15, ozonized air is uniformly sprayed into the cycled air stream and ozone is thoroughly mixed with it.

Referring again to Figure 2, in the next stage, the ozone treated air stream is passed from diffuser 15 to the oxidation catalyst zone 25 which comprises a porous bed or beds of particulate oxidation catalyst(s). The oxidized treated air stream is then fed to the catalytic ozone destruction zone 27. This too is a porous bed or beds of particulate catalyst(s). The air stream that is recovered from this last zone is lower in pollutants content than the incoming air stream to opening 10 and lower in ozone content than the oxidized air stream withdrawn from oxidation catalyst zone 25.

The more purified air stream is then removed from duct 11 through opening 12 into the remaining portion of the duct work comprising the air circulation system in the confined area.

Located in duct 11 is the opening to pipe 29 for feeding cycled air to the ozone generator 21. It may prove useful in some circumstances to add a circulation fan or compressor to pipe 29 to assure supply of air to the ozone generator. Also provided at the end of duct 11 is an ozone monitor 31 of a conventional commercial design suitable to monitor the toxicity level of ozone in the cycled air stream taken from duct 11. The monitor can be coupled to ozone monitor 33 and data from each can be automatically imputted to a computer 35 that is fitted to control the output of ozone generator 21 so that if the level in the air stream exceeds that permissible for the confined area then the ozone levels being generated by the ozone generator can be reduced (or shut off if needed) to reflect safe levels of ozone in the outlet air stream.

Figure 4 is a schematic plan illustration of a laboratory apparatus assembly for the assessing the treatment of air in a recirculating fashion utilizing ozone treatment and simulating in a significant manner the conditions of large scale commercial usage of the process of the invention. The purpose of the assembly is to create, on a small scale, conditions that reflect the effectiveness of the whole process of the invention and any part of the process in order to enhance selection of various types of catalysts, combinations of catalysts, concentrations of certain pollutants that can be treated, measurements of the effectiveness of ozone treatment, and the like considerations. House air is carried into the apparatus assembly through line 101 and then paced through $CO_2$ scrubbers 105, through an oil trap (not shown), and then through a dryer 109 to remove water and lower the relative humidity to, e.g., a dew point of -50 ° C. or less. The air stream is divided into three streams. One stream of the air is fed via line 117 to the bank of reactors labeled 161, 163 and 165. A second stream of the air is used to form the humidified feed and is split into two streams, 204 and 206. Stream 204 is fed to humidifier 203 which is a column of water, provided with water via inlet 205. The humidified stream is recombined with stream 206 to form stream 209. Vent control 207 is used to vent the humidifier upon introduction of water into the humidifier. The first and second streams of the air are eventually treated to add the pollutant feed, typically a hydrocarbon or universal pollutant for general applicability of the process (which is generally acetone). This is accomplished as follows: a tank 113 of the pollutant is put on-line to provide streams 133 and 211. Stream 113 adds a monitored amount of pollutant to

air stream 119 which thereafter becomes polluted stream 137. Stream 211 adds a monitored amount of pollutant to humidified stream 209, which thereafter becomes moist polluted stream 135. Stream 137 feeds reactors 161, 163 and 165. Stream 135 feeds reactors 145, 147 and 151. The third stream of the incoming air is sent to the ozone generator 121 (possessing cooling water inlet 123 and outlet 125) via line 115. Ozone laden air from the ozone generator 121 is split into streams 125 and 129. Stream 125 feeds a monitored amount of ozone into stream 135 via line 127 and stream 129 feeds a monitored amount of ozone into stream 137 via line 131 so that ozone is mixed with the pollutant containing air stream. These ozone/pollutant/air stream are passed through the reactors.

For example, stream 135 is ozone laden via stream 127 and the combined stream 139 is used to feed reactors 145 (via line 141), 147 (via line 143) and 151 (via line 149). The effluent from reactor 145 is combined via line 167 with the effluent from reactor 147 via line 169, and the effluent from reactor 151 via line 171 and vented via line 175. The effluent from reactor 147 may or may not be combined with the effluent from reactor 145, and such may or may not be combined with the effluent from reactor 151, via line 171 and 169, as the case may be. The individual streams may be isolated to be selectively sent through line 173 to the gas chromatographic analyzer maintained onstream with the assembly.

The same principles are followed in the operation of reactors 161, 163 and 165. Ozone containing air is fed via line 153 into these reactors and the effluent streams 177, 179 and 181 from the reactors are matched or mixed or taken off individually, as characterized above so that one can selectively choose an operating procedure and conditions and also have options as to the concentrations and volume of materials. Eventually the effluent from each reactor ends up in the gas chromatographic analyzer to determine the effectiveness of the operation. In addition, each of the effluent lines from the reactors can be fed to an ozone analyzer if the air stream(s) are to be vented. Provisions are made to have the inlets to the reactor feed material to the gas chromatographic analyzer.

With this mode of operation, the two banks of reactors may have different feeds; one may be humidified and one may be dry. These banks could have slightly different pollutant and ozone concentrations since each additive stream is split to provide a feed for both banks of reactors.

The system may also be operated with identical feeds for both banks. To achieve this mode of operation, valve **V1** is closed and valve **V2** is opened. The entire flow for both banks passes through flow meter **FW8**. If desired, this air stream may be humidified. Only one pollutant stream and one ozone stream is then required, and the resultant ozone/pollutant/air stream is split after total mixing to provide identical feeds for both banks of reactors. In this case, lines 131 and 133 are closed.

Samples may be withdrawn throughout the system at ports labeled **SD** or **SW** for manual sample collection. Automatic data collection can occur immediately before and after the reactors as shown in the diagram with the stream labeled 'to GC'. Samples may also be withdrawn and sent to the ozone monitor to determine the ozone concentration before and after the reactors. The ports for the ozone concentration determination after the reactors connect to a 7 port valve 187 which has 6 inlets from the reactors and one outlet which goes to the monitor 196 via line 193. Reactor feed streams may be sent to ozone monitor 196 by closing **V4** and opening either **V3** or **V5**. The ozone monitor 196 requires at least 1 l/min. Any excess air flow is passed through line 197, bubblers 199 and vented via line 201.

The laboratory apparatus system was constructed of 6.35 mm (1/4 inch) OD stainless steel tubing with Swagelok fittings. The reactors are made of 0.95 cm (3/8 inch) OD stainless steel tubing and are 17.8 cm (7 inches) long. The catalysts are held in place in the reactors by a screen at the bottom of the reactors. The humidifier is a 1.2 m (4 feet) long 15 cm (6 inches) ID glass column. It is half filled with distilled water (1 liter). Air passes through a glass frit filter and up through the water and mixes with dry air to produce humidified air. The air passing through the humidifier is at 100%RH and the dry air is 0%RH. The humidity of the resultant air stream is a weighted average of the two streams.

The manual sample ports are Swagelok tees connecting two pieces of tubing with a septum in the third port for sample collection. A 3.2 mm (1/8 inch) ferrule is placed inside the cavity of the fitting to hold the septum in place. This septum is replaced after approximately ten samples have been collected from that port.

House air is dried by a Puregas Heatless Dryer. It supplies up to 1 SCFM of air at 0.62 MPa (90 psi) and -51 ° C. dew point.

The ozone generator is model number T-816 from Polymetrics, Inc. It is rated to produce up to 226 g (0.5 pounds) of ozone per day with a gas flow rate of 0.24 SCFM (1.653 wt %), a voltage of 115 volts, and a pressure of 0.055 MPa (8 psi). Since the ozone/air stream is diluted to obtain lower ozone concentrations needed in the reaction system, a lower voltage is used to generate slightly lower ozone concentrations. The voltage is usually maintained at 90 volts and the air flow rate through the generator at about 4.5 liters/min with a pressure of 0.059 MPa (8.5 psi). The ozone concentration from the generator has a direct relationship

to the air flow rate through the generator. To maintain a constant supply of ozone to the system, the flow rate of air to the system is much less than the total air flow through the generator. A total flow through the generator is about 4.5 liters/min of which only 0.5 liter/min is used by the reaction system. The remainder of the ozone in the air stream can be decomposed and vented to the atmosphere.

The ozone generator is cooled by a chiller from Neslab Instrument Inc., Portsmouth, NH, model Coolflow 33, and is operated at 15°C. The chilling fluid is a 50/50 mixture of distilled water and propylene glycol. The flow rate is maintained at 38 ℓ/h (10 gallons/hour).

The ozone concentration is monitored with an OREC model #03DM-100 from Ozone Research and Equipment Corporation, Phoenix, AZ. It can measure ozone concentration from 0.01 ppm to 1400 ppm and requires a flow of at least 1 liter/min. The ozone measurement system is based on the adsorption of ultraviolet light by ozone as described by the Beer-Lambert Law in conjunction with standard gas laws:

$$I = I_o \exp[-LCX(273/T)(P/760)]$$

where

| | |
|---|---|
| I = | Light intensity with ozone |
| $I_o$ = | Light intensity without ozone |
| L = | Pathlength of absorption cell |
| C = | Ozone concentration |
| X = | Absorption coefficient for ozone at 254 nm |
| T = | Sample temperature, °K |
| P = | Sample pressure, mm Hg |

This method of measurement has been proven to be accurate for both low and high concentration measurement and provides numerous advantages over chemical ozone monitoring techniques, primarily the non-introduction of the analytical chemicals into the sample.

GC analysis of the streams is obtained using a Vartian model 3500 capillary gas chromatograph with a FID detector. The column is a porous layer open tubular polymer (Poraplot® Q) coated fused silica column from Chrompack. The column is 25 m long with a 0.32 mm ID. The typical GC analysis was obtained with an initial column oven temperature of 100°C., ramping to 180°C. at 10°/min. and holding that temperature for nine minutes.

Samples are sent to the GC via a stream selector. This stream selector consists of eight three-way solenoid valves (one alter each of the six reactors and one feed from each bank) and a computerized sequence for selectively opening solenoid valves. The solenoid valves are normally closed and allow the air stream to flow from the reaction system to the vent. At a specified time, a solenoid valve is activated to allow the air stream to flow to the GC. The samples travel from the reaction system to a Valco Valve with the GC which has a 1 ml sample loop. Twenty minutes are allowed for the sample to purge the lines and the sample loop before the sample is automatically injected.

Pressure regulators (**PR6** and **PR7**) are provided installed to equalize the pressure drop through the vent and the GC. Without these regulators, the air would tend to flow through the vent instead of to the GC.

Flow regulators are provided prior to the ozone monitor to regulate the flow going into the monitor. If the pressure were not controlled, it would be easier for the air stream to flow through the monitor than over the catalyst bed, creating a higher flow through the monitor than would normally be seen by the system.

Artificial indoor air is prepared separately. A cylinder of air is mixed with a desired quantity of an organic compound with the balance being air. This cylinder is fed to the reaction system a the "hydrocarbon"/air feed. The cylinders are typically prepared with 0.3 mol % "hydrocarbon" and a final pressure of 0.86 MPa gauge (125 psig).

*Standard Operating Procedure*

Upon receipt of a gas cylinder, a 1 ml cylinder sample is injected manually into the GC to determine the exact concentration of "hydrocarbons" present in the feed. The following are the steps involved in the operation of the indoor air abatement reaction system.

- ♦ Load the catalyst into the reactors. Typically 5g of catalyst is used.
- ♦ Turn on the air and the air dryer.
- ♦ Adjust the air flow rates. Regulator **PR3** regulates the air pressure to the inlet of the ozone generator and is typically set at 0.14 MPa gauge (20 psig). The maximum outlet pressure from the ozone generator is 0.055 MPa gauge (8 psig) and an inlet pressure of 0.14 MPa gauge (20 psig) ensures that minor pressure fluctuations are not seen by the ozone generator. **PR1** and **PR2** regulate the

pressure of the air fed to the two banks of reactors for the system. The regulators are adjusted such that the pressure immediately before the reactors is less than the outlet pressure of the ozone generator to ensure flow from the ozone generator. The exact pressure will vary depending on the type of catalyst in the system and the resultant pressure drop but a typical value for both **PR1** and **PR2** is about 0.069 MPa gauge (10 psig). If all of the reactors are operated under identical conditions, then regulator **PR2** is set to zero. If the two banks of reactors are operated under different conditions, a slightly higher pressure would be set on **PR1** to allow for the increased pressure drop through the humidifier. The flow rates through the reactors should be adjusted until the desired space velocity is reached for the amount of catalyst used in the reactors. This is achieved by adjusting flow meters **FW1, FW2, FW3, FD1, FD2,** and **FD3.**

- Turn on the chiller to start the coolant flow through the ozone generator. Set the temperature (15 °C) and the flow rate (10 GPH) of coolant through the system to desired levels.
- Turn on the ozone generator. After making sure that the power control knob is turned to low voltage, turn on the ozone generator. (Turning the ozone generator on with the power knob at higher voltages may cause problems for the ozone generator.) Adjust the power knob of the ozone generator until a desired voltage reading is obtained. The maximum value is 115V to produce the maximum ozone concentration, and a typical setting is around 90V. The ozone concentration is adjusted in the system at flow meters **FW5, FW6, FD5,** and **FD6.** Flowmeters **FW5** and **FD6** are used for lower ozone flows. Typically the ozone concentration in the air stream before the reactors is monitored and the flow meters are adjusted to achieve the desired ozone concentration.
- Open the valve on the hydrocarbon cylinder and adjust the cylinder's pressure regulator (**PR5**) to a pressure somewhat higher than is needed in the system. A value of 0.14 MPa gauge (20 psig) was typically used. Adjust flow meters **FW4** and **FD4** until the desired concentration of hydrocarbon is reached.
- Turn on the automatic sample collection device--in this case this was controlled by a computer connected switch. This device selects which stream is to be sampled and sent to the GC. The time for the GC to complete a scan is 17 minutes, and samples are changed every 20 minutes. This allows enough time for the lines to completely flush out before the next sample is injected.

The system is then set to run. The first few samples out of the GC are monitored to determine whether the concentration of hydrocarbons and ozone are acceptable. If the hydrocarbon concentration is too low, no peaks will be evident on the GC. If too much ozone is used, a similar situation will occur but only for those hydrocarbons which are known to be susceptible to a homogeneous reaction with ozone. A typical run lasted about 3 days. However, when a scoping experiment was performed, the acceptability of a catalyst was usually evident after a few hours.

Flow readings and ozone concentration readings should be taken every hour for the first few hours to determine the stability of the system. Readings are then taken twice a day.

*Testing Different Types of Supports*

The bare supports, LZ-10, LZ-20, and silicalite, show adsorption characteristics for some of the hydrocarbons utilized as pollutants in an air stream, prior to the addition of ozone to the catalyst bed systems. With a 30% relative humidity air feed, benzene was adsorbed slightly on the silicalite and not at all on the LZ-10 and LZ-20 supports. The butane was not adsorbed at all on all three supports. With a dry air feed, benzene was adsorbed somewhat on both the silicalite and the LZ-10 supports while the butane was not adsorbed at all.

When ozone was added to the system, benzene was eliminated below the detection limits of the instruments, about 1 ppm, with a dry air feed for all three supports. Table 1 shows the percent of benzene and butane remaining in the outlet air stream compared to the inlet conditions for the three supports under both dry and wet conditions. Silicalite outperforms the other supports for the decomposition of hydrocarbons in the presence of ozone. All reactors were treated with the same feed.

Table 1

| SUPPORT | BENZENE DRY | BENZENE WET | BUTANE DRY | BUTANE WET |
|---|---|---|---|---|
| silicalite | 0 | 26 | 8 | 61 |
| LZ-10 | 0 | 67 | 26 | 78 |
| LZ-20 | 0 | 64 | 20 | 74 |

The same supports, silicalite, LZ-10 and LZ-20, were provided with a cobalt/molybdate catalyst (4% CoO, 27.7% $MoO_3$) by the aforementioned impregnation procedure. The various supported catalysts were tested under both humidified and dry conditions. The initial ozone concentration was on the order of 700 ppm $0_3$ with space velocities (defined as volume of feed per volume of catalyst) of 600 $min^{-1}$. The feed to the reactors consisted of ethane, acetone, and benzene. These tests showed that this catalyst loaded LZ-20 oxidized more of the hydrocarbons than did either the catalyst loaded LZ-10 or the silicalite though all three showed activity in oxidizing the hydrocarbon with ozone.

*An Examination Of Some Silicalite Supported Metal Catalysts*

The following silicalite supported catalysts were prepared:

- 28%$MoO_3$, 3%CoO, 5%CuO loaded catalyst
- 1.3%CrO, 2.7%$MoO_3$, 0.5%$Fe_2O_3$, 1%NiO, 1%CoO, 2%CuO loaded catalyst
- 1.3%CrO, 2.7%$MoO_3$, 0.5%$Fe_2O_3$, 1%NiO, 1%CoO, 5.4%$WO_3$, 2%CuO loaded catalyst

Each catalyst was tested utilizing an air stream at a space velocity of 550 $min^{-1}$ having a 25% relative humidity, containing an inlet ozone concentration of 650 ppm and a 10 ppm inlet "hydrocarbon" feed concentration of each of ethane, benzene, and acetone.

The benzene was removed entirely due to homogeneous reaction with ozone. The acetone was also removed entirely by the ozone under both wet and dry conditions after having passed over the catalyst. The ethane was best reduced by the tungsten free catalyst though the tungsten containing catalyst was more effective than the catalyst which only contained cobalt/molybdenum/copper. All of the catalysts performed better under dry conditions.

Five light loadings of metal ions on silicalite were also investigated:

- 0.83%CrO, 0.16%CoO loaded catalyst
- 0.83%$WO_3$, 0.15%CoO loaded catalyst
- 0.84%$MoO_3$, 0.60%$Fe_2O_3$ loaded catalyst
- 0.84%$MoO_3$, 0.15%NiO loaded catalyst
- 0.84%$MoO_3$, 0.15%PdO loaded catalyst

In each experiment, the air stream, having a 25% relative humidity, was fed at a space velocity of around 550 $min^{-1}$, and the inlet ozone concentration was 900 ppm. The "hydocarbon" feed was an equimolar ratio of benzene, acetone, ethanol, methanol, propane, and ethylene. Immediately before the reactors, the acetone and propane concentrations were approximately 10 ppm while the benzene was about 5 ppm. The other hydrocarbons in the mixture were homogeneously reacted to $CO_2$ and water before reaching the reactors. With these light loadings of metals onto the surface of the silicalite, very little oxidation was observed; less than 5% of the remaining hydrocarbons were oxidized. It is thus observed that there is a correlation between oxidation rate and catalyst loadings; higher loadings effect higher oxidation rates.

Higher loadings of certain metal ions on silicalite were tested. A 3% CoO and 16% $MoO_3$ base was used and around 5% of other metal oxides were added:

- 3%CoO, 16%$MoO_3$, 5%$Y_2O_3$ loaded catalyst
- 3%CoO, 16%$MoO_3$, 4%PdO loaded catalyst
- 3%CoO, 16%$MoO_3$, 5%NiO loaded catalyst
- 3%CoO, 16%$MoO_3$, 5%CuO loaded catalyst
- 3%CoO, 16%$MoO_3$, 7%MnO loaded catalyst
- 3%CoO, 16%$MoO_3$, 5%CrO loaded catalyst
- 3%CoO, 16%$MoO_3$, 8%$Ce_2O_3$ loaded catalyst
- 3%CoO, 16%$MoO_3$, 7%$Y_2O_3$ loaded catalyst

These catalysts were tested at three ozone concentrations, 900, 500, and 100 ppm. Oxidation of the "hydrocarbons" by ozone was effected in the presence of all of these catalysts. However, in these tests, the

addition of manganese and/or chromium to the support enhanced catalytic performance. At low ozone concentrations, the addition of cerium or chromium resulted in an increase in the oxidation rate of acetone and heptane.

A long term study was performed to determine catalyst life for these catalysts. In the study, each catalyst saw more hydrocarbon than would be seen in over a year at indoor air concentrations. No decrease in activity was exhibited by any of the catalysts over the test period.

Another set of the catalysts were made, except in this case, the solvent solutions used in impregnating the catalytic material onto the support was a 50/50 methanol/acetone mixture and such were contrasted with the same catalyst concentrations made with water as the solvent to see if the solvent used in the catalyst preparation plays a significant role in the activity of the catalyst. It was determined in this case that the activity of the catalyst was not materially affected by the choice of the solvent.

*Evaluation Of Some Commercially Available Oxidation Catalysts*

Harshaw's copper chromite catalyst, both the tabular form and the extrudate form, and Harshaw's titanium catalysts were tested in the reactors. Space velocities of 550 $min^{-1}$ and an initial ozone concentration of 650 ppm were used. None of the ethane was oxidized by the ozone in the presence of these catalysts. However, some of the acetone was oxidized. Under dry conditions, less than 50% of the acetone was oxidized by the copper/chromite in the tablet form while more than 50% was oxidized in the presence of the extrudate form. In the presence of the titanium catalysts, more than 50% of the acetone was oxidized. None of the catalysts performed well when the air was humidified. Of these three, the copper/chromite extrudate performed better than the others.

Harshaw's Co/Mo catalyst and 0.5% Pd catalyst, both deposited on $\alpha$-alumina, and an Englehard version of a copper chromite were similarly tested. Butane and ethane were the markers in determining catalyst performance. None of the catalysts reduced the concentration of either ethane or butane by more than 5% under both wet and dry conditions. The space velocity was 600 $min^{-1}$ and the initial ozone concentration was 600 ppm.

*Custom Catalyst Developments*

A designed experiment was carried out to evaluate eleven metal ions, Mn, Co, Fe, Cu, Ni, Zn, Mo, Ce, Cr, Pd, and W, that had been identified as potentially suitable for use in commercially effective catalysts for the oxidation of VOC's and biologicals in the presence of ozone. The twelve metal ions were placed on the surface of supports and their respective concentrations, as set forth (as added basis) in Table 2, were varied simultaneously and in a random fashion.

## Table 2

### Weight Percents Of Metal Ions On The Surface Of Catalyst Support

| run | Co | Cr | Cu | Fe | Mn | Mo | Ni | Zn | Pd | W |
|---|---|---|---|---|---|---|---|---|---|---|
| 1 | 0.4000 | 0.2000 | 0.6800 | 0.3900 | 2.9000 | 0.3200 | 1.6000 | 0.4100 | 0.0630 | 0.5000 |
| 2 | 0.6300 | 0.3100 | 0.5300 | 0.3400 | 2.2600 | 0.4000 | 1.7000 | 0.2770 | 0.1000 | 2.0000 |
| 3 | 1.3400 | 0.8400 | 0.4100 | 0.3480 | 2.6100 | 0.1500 | 0.3800 | 0.7150 | 0.2400 | 0.5400 |
| 4 | 0.8220 | 0.7720 | 0.9370 | 0.3930 | 2.9200 | 1.0500 | 0.1540 | 0.9050 | 0.2000 | 0.9700 |
| 5 | 0.9800 | 0.5500 | 0.0400 | 0.2000 | 3.8000 | 0.8200 | 0.4600 | 0.0880 | 0.1200 | 0.2600 |
| 6 | 0.2400 | 0.0860 | 1.1600 | 0.3100 | 3.1500 | 1.4000 | 2.6100 | 0.7650 | 0.0250 | 0.3600 |
| 7 | 0.7550 | 0.6020 | 1.4200 | 0.4200 | 1.6400 | 0.0892 | 2.4100 | 0.2700 | 0.0490 | 0.6000 |
| 8 | 0.1950 | 0.6430 | 1.7600 | 0.5470 | 3.8900 | 1.7800 | 0.3930 | 0.2460 | 0.0800 | 0.3940 |
| 9 | | | | | | | | | | |
| 10 | 0.9820 | 0.2980 | 0.3070 | 0.2010 | 2.0900 | 0.7000 | 0.1820 | 0.5650 | 0.3300 | 0.4100 |
| 11 | 0.3600 | 0.5600 | 2.4000 | 0.5000 | 1.7000 | 2.0000 | 0.2400 | 0.5400 | 0.3000 | 1.0000 |
| 12 | 0.3640 | 0.2980 | 0.0614 | 0.2080 | 3.5500 | 0.7000 | 1.1200 | 0.4190 | 0.1300 | 0.0740 |
| 13 | 0.7940 | 0.3030 | 0.3200 | 0.5490 | 3.9000 | 0.1990 | 0.2700 | 0.7360 | 0.0070 | 0.8500 |
| 14 | 0.7870 | 0.6800 | 0.9290 | 0.2000 | 3.9000 | 0.0701 | 0.4750 | 0.4920 | 0.1200 | 0.3350 |
| 15 | 0.7550 | 0.4150 | 1.3000 | 0.4380 | 1.3700 | 0.8380 | 1.6400 | 0.5570 | 0.0050 | 1.0300 |
| 16 | 0.4100 | 0.5940 | 1.6000 | 0.2320 | 0.1960 | 0.4400 | 0.8750 | 0.9000 | 0.2000 | 0.5300 |
| 17 | 1.0000 | 0.2000 | 2.0000 | 0.3000 | 0.2000 | 0.2000 | 0.7000 | 0.2000 | 0.2000 | 0.0200 |
| 18 | 0.8100 | 0.4900 | 0.4490 | 0.3900 | 1.3000 | 1.2000 | 1.8000 | 0.3120 | 0.1600 | 0.3400 |
| 19 | 0.5000 | 0.5500 | 0.6270 | 0.7270 | 3.5000 | 0.0950 | 0.2600 | 0.5100 | 0.2100 | 0.3530 |
| 20 | 0.4380 | 0.5250 | 0.3300 | 1.7000 | 2.7500 | 2.3000 | 0.3220 | 0.7540 | 0.2000 | 0.8800 |
| 21 | 0.5900 | 1.7100 | 0.9800 | 1.2200 | 9.3000 | 0.6980 | 0.5000 | 0.1700 | 0.3700 | 1.1000 |
| 22 | 2.4000 | 0.6200 | 1.6000 | 0.3000 | 3.7000 | 3.0000 | 3.2000 | 0.0820 | 0.4000 | 2.0000 |
| 23 | 0.7760 | 1.0100 | 2.2400 | 1.2600 | 8.6300 | 1.7500 | 2.1200 | 2.1000 | 1.3000 | 3.9000 |
| 24 | 0.2300 | 0.5100 | 2.2000 | 0.8000 | 5.8000 | 1.1000 | 4.5500 | 1.5200 | 0.4700 | 1.0000 |
| 25 | 2.5000 | 1.3000 | 1.7000 | 0.7000 | 1.3000 | 3.0000 | 5.0000 | 0.9200 | 1.0000 | 2.0000 |
| 26 | 5.9386 | 1.0175 | 4.5538 | 0.8549 | 3.4994 | 3.3268 | 2.0453 | 1.4883 | 2.4373 | 13.8442 |
| 27 | 2.2945 | 0.1100 | 2.0096 | 1.2189 | 11.4239 | 2.6582 | 3.5734 | 2.4016 | 2.9132 | 4.3339 |
| 28 | 2.2440 | 0.9586 | 4.8430 | 2.1223 | 1.9326 | 0.5881 | 7.6016 | 2.6107 | 0.8476 | 4.6046 |
| 29 | 1.4958 | 1.4565 | 2.9948 | 0.3722 | 5.2856 | 3.2905 | 4.6942 | 1.9905 | 0.0000 | 3.1424 |
| 30 | 2.1249 | 1.7931 | 5.2868 | 0.6568 | 6.8628 | 1.8952 | 6.1705 | 0.6657 | 1.5574 | 2.4672 |

The performance of the catalysts of Table 2 as oxidation catalysts are set forth in Table 3 below.

Table 3

| run | fraction of acetone remaining | fraction of ozone remaining |
|---|---|---|
| 1 | 0.63 | 0.9281 |
| 2 | 0.60 | 0.9162 |
| 3 | 0.57 | 0.8862 |
| 4 | 0.472 | 0.8626 |
| 5 | 0.52 | 0.8626 |
| 6 | 0.422 | 0.8462 |
| 7 | 0.605 | 0.8056 |
| 8 | 0.458 | 0.8056 |
| 9 | | |
| 10 | 0.405 | 0.6333 |
| 11 | 0.523 | 0.7833 |
| 12 | 0.562 | 0.7222 |
| 13 | 0.781 | 0.8158 |
| 14 | 0.69 | 0.7895 |
| 15 | 0.59 | 0.7737 |
| 16 | 0.609 | 0.7684 |
| 17 | 0.517 | 0.7409 |
| 18 | 0.423 | 0.7979 |
| 19 | 0.428 | 0.8446 |
| 20 | 0.585 | 0.8549 |
| 21 | 0.638 | 0.8653 |
| 22 | 0.35 | 0.7659 |
| 23 | 0.446 | 0.7883 |
| 24 | 0.124 | 0.6168 |
| 25 | 0.116 | 0.5794 |
| 26 | 0.186 | 0.6542 |
| 27 | 0.453 | 0.5199 |
| 28 | 0.116 | 0.5204 |
| 29 | 0.266 | 0.7704 |
| 30 | 0.207 | 0.7459 |

The experimental results (though added, Ce was not analyzed for) showed that four metals, Mo, Ni, Pd, and W, showed superiority in oxidizing the hydrocarbons by ozone. Catalysts comprising these four metal ions only were made and tested. It was found that this combination did perform as expected with over 80% of the VOC's present being oxidized, at least initially.

Catalyst 9 was liquid alter molybdate, manganese and cobalt were added. However, upon adding the iron, the mixture became solid. A second attempt was made adding the iron immediately alter the molybdate but this also formed a solid. Catalyst 9 was thus eliminated from the study. All of the other catalysts were prepared according to the general catalyst preparation procedure set forth above with the following notations and exceptions:

*Catalyst Preparation*

For each catalyst preparation run, the specified metal ions were dissolved in 7 ml of water. Ammonium molybdate was added first because it was difficult to dissolve. Due to the relatively large number of metal ions in the study, the final solution was not homogeneous. Tungsten did not dissolve in water so it was present as suspended particles in all of the runs. The support used in each run was silicalite.

The specific technique for adding the metal ion mixture to the support was as follows:

- ♦ Place 20 g of silicalite into a flask under house vacuum for at least half an hour.
- ♦ Add the specified metal salts to 7 ml water.
- ♦ Remove approximately 7 ml of the metal ion solution with a syringe and inject onto the support. Additional solution could be added depending on the quantity of solution which was adsorbed by the support
- ♦ Continue vacuuming for about half an hour.

- ♦ Remove the impregnated support from the flask and dry overnight at 100°C.
- ♦ Calcine the dried support at 400°C. overnight with air flowing over the catalysts.

The addition of the metal salts to 7 ml of water resulted in a total solution volume greater than the pore volume of the support. Consequently, the total solution exceeded that which could be adsorbed by the support. For the first nineteen catalysts, a total of 7 ml of the metal salt solution was added to the support. After the twentieth catalyst the entire solution was added to the support. Since the pores were filled after the addition of the first addition of solution, addition of more solution required subsequent addition steps after the first addition of the metal salt solution, drying at 100°C. overnight and then calcining overnight at 400°C. The final catalyst step following the last addition was drying and calcining.

*Experimental Parameters*

The catalysts were each loaded into the reactors discussed above. Identical flow conditions and concentrations were used throughout the entire set of designed experiments. Acetone was used at the marker to indicate the extent of the oxidation of the hydrocarbons. The concentration of acetone immediately before the reactor, was set to 10 ppm; while the ozone concentration immediately prior to the reactor was 200 ppm. The hydrocarbon feed to the reaction system contained other components and these feed concentrations are as follows:

| Runs | Feed Component | Feed Composition mol % |
|---|---|---|
| 1-21 | heptane<br>benzene<br>acetone | 0.3<br>0.3<br>0.3 |
| 22-30 | ethanol<br>methanol<br>acetone<br>propane<br>ethane | 0.3<br>0.3<br>0.3<br>0.3<br>0.3 |

The air passing through the reactors had a space velocity of 500 volumes of feed per volume of catalyst per minute and was maintained at a relative humidity of 25%. It was found that most catalysts which were active for oxidation of hydrocarbons had a initial unsteady-state period during which their activity was greater than the steady-state condition. Testing of each catalyst was continued until a steady-state situation was achieved.

*Results*

Each catalyst was tested for a period of three days. The steady-state fractions of acetone remaining after the air streams passed over the catalysts are given in Table 3 for each catalyst. Also given in this table are the final ozone concentrations at the outlet of the reactors obtained at the end of the three day period.

These results indicate that the type of catalysts mixture does make a difference in the ability of the ozone to oxidize the hydrocarbons which might be present in the air stream. Depending on the catalyst mixture, the percent of acetone which was oxidized ranged from 20% to 80%.

*Hydrocarbon Destructability*

The results of the designed set of experiments were analyzed for statistical significance. Based on the correlation matrix, all of the metals had a negative effect on the responses, that is, the presence of each metal ion caused a reduction in the acetone and ozone concentrations. However, the only metals which had a significant influence, in the range of the data, were Mo, Ni, and Pd. In addition, there was a strong correlation between Pd and W which caused W to enter the models. Tungsten entered the models as a positive term even though its original correlation coefficient was negative.

The response, defined as the fraction of acetone remaining alter the catalyst beds, was found to be:

$$Y1 = 0.626 - 0.056*Mo - 0.06*Ni - 0.321*Pd + 0.112*W$$
$$R^2 = 0.752$$

RSD = 0.085

with the metal ion concentrations given in terms of weight percents. The results suggest that a good catalyst would contain high levels of Mo, Ni and Pd and a low level of W.

The statistical model predicts that only four of the metal ions evaluated played major catalytic roles in aiding the oxidation of hydrocarbons by ozone. Six catalysts were prepared with various mixtures of these metals to confirm the model. The weight of metal salts added to the solution and the calculated weight percents are given in Table 4 along with the fraction of acetone remaining after passing over the catalyst. After the test period of three days, five of the six catalysts prepared allowed over 80% of the acetone to be oxidized. These catalysts also showed an outlet propane concentration higher than the inlet while the ethane concentration was equal to the inlet.

The catalysts were allowed to continue in service after the test period. During this period, only dry air was passed over the catalyst beds and the ozone and the hydrocarbon streams were shut off for a period of twelve hours. After the streams were returned to normal operating conditions, it was found that the activity of the catalyst had decreased. The activity level had decreased so that only 50% of the acetone was oxidized instead of 80% as seen before. However, this 50% activity level remained constant for over three days.

*Ozone Destructability*

Some of the "hydrocarbon" destruct catalysts also reduced the ozone concentration below expected levels in addition to aiding the oxidation of "hydrocarbons." A relationship was found between certain metal ions in solution to the final ozone concentration. The response, defined as the fraction of ozone remaining alter the reactors, can be correlated by the following equation:

Y2 = 0.827 - 0.029*Ni - 0.25*Pd + 0.08*W
$R^2$ = 0.553
RSD = 0.063

with the concentration of metal ions given in terms of weight percents. The correlation coefficient ($R^2$) value is not as good as it was for the response for the decrease in the acetone concentration. Also, the metals which were found to be important in reducing the ozone concentration are the same ones which were found to be important in reducing the acetone concentration. This is logical since acetone is reduced by reaction with ozone. It is therefore expected that the catalyst which causes the largest reduction in acetone to also have a significant reduction in ozone as well.

Table 4

| | g metal salt added | | | | wt % metal ion | | | | fraction of acetone remaining |
|---|---|---|---|---|---|---|---|---|---|
| run | Mo | Ni | Pd | W | Mo | Ni | Pd | W | |
| 1 | 0.0 | 0.0 | 0.6465 | 0.0 | 0.0 | 0.0 | 1.47 | 0.0 | 0.8 |
| 2 | 0.0 | 13.6403 | 0.0 | 0.0 | 0.0 | 12.10 | 0.0 | 0.0 | 0.098 |
| 3 | 2.0014 | 13.6416 | 0.0 | 0.0 | 4.56 | 11.55 | 0.0 | 0.0 | 0.071 |
| 4 | 2.0030 | 13.6413 | 0.6475 | 0.0 | 4.51 | 11.41 | 1.24 | 0.0 | 0.068 |
| 5 | 2.0112 | 13.6445 | 0.6492 | 1.0025 | 4.39 | 11.07 | 1.20 | 2.96 | 0.099 |
| 6 | 2.0047 | 6.0309 | 0.3090 | 1.0151 | 4.70 | 5.25 | 0.62 | 3.22 | 0.076 |

*Carbon Mass Balance Illustrations*

Table 5 demonstrates the performance of a number of multimetal salts deposited on silicalite as oxidation catalyst with ozone for the oxidation of benzene and butane. Specifically, when comparing hydrocarbon oxidation with $CO_2$ production one sees that nearly 100% of the carbon is converted to $CO_2$ for catalysts 5A and 5B. When silicalite is used as the catalyst, the mass balance does not close as well, showing that the catalytic metal sites are very important.

TABLE 5

| TESTING OF CATALYSTS 5A AND 5B (SEE TABLE 7) AND SILICALITE AS OXIDATION CATALYSTS FOR 4 DAYS | | | |
|---|---|---|---|
| s = 230/min | 5A | 5B | silicalite |
| Day 4 | | | |
| Benzene<br>Butane<br>CO2 Closure | 0.45<br>0.50<br>85% | 0.47<br>0.57<br>89% | 0.26<br>0.40<br>50% |
| s = 570/min | | | |
| Benzene<br>Butane<br>$CO_2$ Closure | 0.63<br>0.62<br>104% | 0.65<br>0.84<br>180% | 0.54<br>0.59<br>70% |

* = output/input

5 = 1 weight % $F_2O_3$, 1 weight % NiO, 1 weight % $CoO_2$, 7.5 weight % $CrO_2$, 7.5 weight % $MoO_3$
6 = 1 weight % $Fe_2O_3$, 1 weight % NiO, 1 weight % $CoO_2$, 5 weight % $CrO_2$, 5 weight % $MoO_3$, 5 weight % $WO_3$
A = salts combined together before loading
B = nitrates added before tungstic acid and ammonium molybdate
C = tungstic acid and ammonium molybdate added before nitrates

*Activity of the oxidation catalyst with typical indoor air concentrations.*

The ability of an oxidation catalyst to reduce hydrocarbon pollution below 20 parts per billion (pbb), the detection limits, was evaluated. Table 6 shows the inlet and outlet concentration of butane where the space velocity air stream containing the butane was about 200/min. This low space velocity was used to ensure that if the catalyst worked at all, then significant differences between the inlet and outlet concentrations would be observed. The catalyst used in the experiment contained 1 weight % CoO, 1 weight % $Fe_2O_3$, 1 weight % NiO, 5 weight % $MoO_3$, 5 weight % $WO_3$ and 5 weight % $CrO_2$ loaded onto silicalite. In making this catalyst, the tungsten and the molybdenum salts were simultaneously impregnated into the silicalite and the other salts were post added. The data shows that no matter what the inlet concentration of butane, the outlet concentration was below detection limits therefore showing that the catalyst functions efficiently at low hydrocarbon concentrations.

Table 6

| INLET AND OUTLET BUTANE CONCENTRATION | | |
|---|---|---|
| | Inlet | Outlet |
| Run #1<br>Run #2<br>Run #3 | 750 ppb<br>530 ppd<br>120 ppb | <20 ppb<br><20 ppb<br><20 ppb |

*Other Ozone Decomposition Catalysts*

A simple ozone decomposition catalyst was formed by placing about 0.5 gram of glass wool in a container to which was added 1 gram of platinum black, closing the container and shaking the container to disperse and adhere the platinum black to the glass wool. The supported platinum black was used as an ozone decomposition catalyst by passing an air stream containing 10 ppm of ozone at a rate of 3 liter/min. and at 95 m/min. (290 ft/min.) over the Pt catalyst. The ozone concentration dropped to about 10-30 ppb and maintained over a period of 7 days.

*Oxidation of Halogenated Hydrocarbons*

As pointed out previously, halogenated hydrocarbons are VOC's associated with sick building syndrome. A feature of this invention is the ability to oxidize halogenated hydrocarbons to $CO_2$, water and an adsorbed halide species so that halogen is not recycled back to the building with the recycled air stream. The following test showed that halogenated hydrocarbons can be effectively oxidized and the separated halogen is sequestered by the catalyst system and kept from being recycled with the air stream.

In this study, the catalysts tested were silicalite 1.59 mm (1/16 inch) extrudate, Carulite® [a mixture of $MnO_2$, CuO, and $K_2O$ (40%Mn, 9%Cu, 1.2%K and 5.3% Al)], an industrial ozone decomposition and oxidation catalyst, and a mixed metal oxidation catalyst (6-8-S115) consisting of 1 weight % $Fe_2O_3$, 1 weight % NiO, 1 weight % $CoO_2$, 5 weight % $CrO_2$, 5 weight % $MoO_3$, 5 weight % $WO_3$, all loaded onto silicalite as the support. (This catalyst was prepared by loading the metal nitrates onto silicalite while the silicalite was under a partial vacuum. It was then dried at 100°C. overnight and calcined at 400°C. overnight.) The halohydrocarbon that was tested was perchloroethylene ("PCE").

In each run, throughput in the reactor of the feed air stream was carried out prior to ozone addition to the air stream to ensure that the inlet and outlet concentrations of PCE were the same. Only when the outlet was equal to the inlet was the ozone flow turned on. In this way adsorption effects were accounted for. When the ozone was turned on the concentration of PCE in the outlet from the reactors droppped and then slowly rose. Due to experimental error, the calculated loadings were determined to be off by no more than 35%.

Table 7 recited the calculated loadings of chlorine and the measured loadings. The amount of chlorine that should have been adsorbed by the catalyst was calculated from the difference between in the inlet and outlet concentrations of PCE and is given as the calculated loading. Runs were made under three different relative humidities: as dry as possible (about 0-5%), 33% and 67%. As the humidity was increased from as dry as possible to a measurable RH, the activity of the catalysts decreased. The data for the 0% and 33% RH are taken at the same space velocity and linear velocity. At 67% RH, the air velocity was slowed down in hopes of seeing some activity with the Carulite®, but to no avail.

In the case of silicalite, when dry air was used with 10 ppm PCE in the feed gas, mass balance calculations show that the loading of chlorine should be about 2.9%. These calculations are based upon the amount of chlorine being consumed by the oxidation of PCE. Analysis shows the catalyst surface loading is 4.7% and the cross-section loading is about 2%. This showed reasonable agreement between the mass balance calculations and the actual analysis indicating that most of the chlorine is adsorbed onto the surface of silicalite. When humid air was employed, the activity of the catalyst decreased, but the chlorine from the oxidized PCE was still adsorbed onto the surface. After the run at 33% RH, the catalyst pulled from the reactor possessed a chlorine-type odor.

Carulite® showed almost no activity in humid air. This agrees quite well with Carus Chemical Co.'s (the supplier of Carulite®) literature stating that chlorine poisons Carulite® when used as an oxidation catalyst. When dry air was used mass balance calculations indicate that about 4.5 weight % of the catalyst should be chlorine. Under humid conditions, analysis showed about 0 weight % chlorine on the catalyst and so did the material balances. Furthermore, Carulite® tested at 33% RN had a chlorine-type smell.

6-8-S115 was the most active of all the catalysts tested. Under dry conditions, however, material balances indicate that 3.7% of the catalyst should be chlorine. Analysis show only 2% chlorine in the surface and 1.3% chlorine in the cross-section. When humid air was used the agreement between the calculated and measured loadings was quite good. 6-8-S115, after the run at 33% RH, had no chlorine-type smell.

Another experimental effort was carried out with the object of comprehending the amount of carbon dioxide generated when perchloroethylene is oxidized by ozone. The following reaction scheme is presumed:

$$M + O_3 + C_2Cl_4 \rightarrow CO_2 + O_2 + M\text{-}Cl$$

where M is the metal surface of the catalyst.

These data suggest that for the indoor air pollution abatement, where small amounts (on the order of tens of ppb) of chlorinated hydrocarbons are oxidized by ozone, highly dangerous chlorinated species should not form.

TABLE 7

| Chlorine Balance Closure | | | |
|---|---|---|---|
| | SILICALITE | | |
| | 0-5% RH | 33% RH | 67% RH |
| Mass Balance<br>Surface<br>Cross-section | 2.88<br>4.7<br>1.96 | 0.94<br>0.88<br>0.1 | 1.74<br>3.45<br>1.67 |
| | CARULITE® | | |
| | 0-5% RH | 33% RH | 67% RH |
| Mass Balance<br>Surface<br>Cross-section | 4.47<br>3.4<br>1.73 | 0.21<br>0.09<br>0.06 | -1.49<br>0<br>0 |
| | 6-8-S115 | | |
| | 0-5% RH | 33% RH | 67% RH |
| Mass Balance<br>Surface<br>Cross-Section | 3.73<br>1.9<br>2.08 | 1.44<br>1.93<br>1.38 | 2.37<br>1.35<br>2.08 |

*Treatment of Biologicals*

To illustrate the use of the process for the reduction of biological aerosols, a test organism was selected that would be distinctive enough to be useful in the experimental work yet would readily reveal the efficacy of the process. The test organism was *Serratia marcescens.* It was chosen because of its distinctive red color. It was also an indoor air pollutant in at least one case. To accentuate its pigment formation, *S. marcesens* was grown on Pseudomonas agar P.(VWR). The bacteria were sampled with the use of an Anderson Sampler. A piece of 2.5 cm (one inch) Tygon® tubing was attached from the sample valve to the top of the sampler. A calibrated vacuum pump drew 0.027 $m^3$ (one cubic foot) per minute through the sampler. Normally, the colony counting procedures have the number of CFU per plate between 30 and 300, however, because in the use of the Anderson Sampler, at above 100 CFU/plate, it was not possible to be certain that only one colony was growing at a specific rate. Therefore, only the results of CFU/plate less than 100 were considered valid.

In these samples, the catalyst was silicalite (1.59 mm (1/16 inch) exudates) ---chosen because of its excellent performance as a support for a number of oxidation catalysts. The air flow was 30,000 m/min, using a 10 gram of silicalite bed, resulting in a space velocity of 3,000/min. (linear velocity of 95.4 m/min.---about 313 ft/min.). The results are set forth Table 8.

Table 8

| Site of Reactor Measurement (inlet or outlet) | Aerosol Particle Size (μm) | Concentration Ozone (ppmv) | | |
|---|---|---|---|---|
| | | 0 | 5 | 10 |
| | | ($CFU/ft^3$) $CFU/m^3$ | | |
| INLET | 1.1 - 2.1 | 1150(31) | 780(21) | 220(6) |
| OUTLET | 1.1 - 2.1 | 780(21) | 260(7) | 37(1) |
| INLET | 0.65 - 1.1 | 1960(53) | 590(16) | 260(7) |
| OUTLET | 0.65 - 1.1 | 1400(38) | 150(4) | 150(4) |

Thus, by the action of the catalyst bed and ozone, a 1 log reduction of the bioaerosol was observed.

## Claims

1. A process for treating indoor air containing pollutants including volatile organic compounds and biological pollutants comprising the following steps:
   (a) withdrawing an indoor air stream from a confined indoor area;
   (b) mixing the indoor air stream of (a) with 0.1 to 1500 ppm of ozone;
   (c) treating the mixture of step (b) in an air treatment zone containing a solid supported oxidation catalyst which comprises a metal selected from iron, ruthenium, osmium, cobalt, rhodium, iridium, nickel, palladium, platinum, molybdenum and tungsten;
   (d) treating the treated mixture of step (c) in an ozone decomposition zone containing an ozone decomposition catalyst selected from nonzeolitic molecular sieves, activated carbon and a supported catalyst comprising a metal selected from nickel, palladium, platinum, manganese, copper and silver so as to obtain a treated air stream containing not more than 0.05 ppm of ozone; and
   (e) circulating the treated air stream of step (d) to the confined indoor area of step (a).

2. The process according to claim 1 wherein the metal of the oxidation catalyst used in step (c) is supported on a molecular sieve of the zeolitic or nonzeolitic variety.

3. The process according to any one of claims 1 and 2 wherein the ozone decomposition catalyst used in step (d) is a supported metal catalyst.

4. The process according to claim 3 wherein the support of the ozone decomposition catalyst is a porous support.

5. The process of at least one of the claims 1 to 4 wherein the temperature and pressure of the air treatment are essentially at ambient conditions.

6. The process of at least one of the claims 1 to 5 which is conducted at ambient relative humidity, ambient temperature and ambient pressure conditions of the air stream undergoing treatment.

7. An apparatus comprising a catalytic depollution zone comprising an ozone catalytic treatment zone and an catalytic ozone destruction zone, located in association with the cyclization of air within a large confined structure, wherein the catalytic depollution zone contains:
   a) a transport means for the passage of an air stream from the large confined structure to the catalytic depollution zone,
   b) a transport means for the passage of the air stream from the catalytic depollution zone to the large confined structure,
   c) an ozone catalytic treatment zone comprising
      i) a source of ozone associated with the ozone catalytic treatment zone,
      ii) an open connection to the transport means for the passage of air from the large confined structure to the ozone catalytic treatment zone,
      iii) a distribution means for mixing ozone with the air introduced to the ozone catalytic treatment zone from the large confined structure,
      iv) a solid porous oxidation catalyst layer located downstream of the distribution means and to which is fed the ozone containing air passed from the distribution means, said oxidation catalyst being a supported catalyst comprising a metal selected from iron, ruthenium, osmium, cobalt, rhodium, iridium, nickel, palladium, platinum, molybdenum and tungsten,
      v) an outlet from the ozone catalytic treatment zone to a catalytic ozone destruction zone,
   d) a catalytic ozone destruction zone comprising
      i) an open connection to the ozone catalytic treatment zone,
      ii) a solid porous ozone decomposition catalyst suitably positioned to make contact with the air stream passed from the ozone catalytic treatment zone to the catalytic ozone destruction zone, said ozone decomposition catalyst being selected from nonzeolitic molecular sieves, activated carbon and a supported catalyst comprising a metal selected from nickel, palladium, platinum, manganese, copper and silver,

iii) an outlet from the catalytic ozone destruction zone to the transport means for the passage of the air stream from the catalytic depollution zone to the large confined structure,

e) ozone monitors positioned to determine the concentration of ozone present in the air stream supplied to and removed from the catalytic depollution zone and means for regulation of ozone supplied to and removed from the catalytic depollution zone.

**8.** The apparatus of claim 7 wherein the catalytic depollution zone apparatus is associated with one or more of the air conditioning and heating systems of the large confined area and it is operated in concert wich such systems.

**9.** The apparatus of at least one of the claims 7 to 8 wherein the forced air blowers of the furnace or the air conditioning systems will control the air flow rate through the catalytic depollution zone.

**10.** The apparatus of at least one of the claims 7 to 9 wherein the catalytic depollution zone is located on the return air or the forced air side of such furnaces and/or air conditioning equipment serving the large confined area and as part of the air circulation scheme of the large confined area.

**11.** The apparatus of at least one of the claims 7 to 9 wherein the catalytic depollution zone is provided on the return air side of the air conditioning and/or heating systems with which it is associated.

**12.** The apparatus of at least one of the claims 7 to 11 wherein a filtration means for the removal of particulates from the air stream is located before or after the ozone distribution means used to mix ozone with the air, and located prior to the solid porous oxidation catalyst layer located downstream of the distribution means.

## Patentansprüche

**1.** Verfahren zur Behandlung von Innenraumluft, welche Verunreinigungen, einschließlich flüchtiger organischer Verbindungen und biologischer Verunreinigungen, enthält, welches folgende Schritte umfaßt:

(a) Abziehen eines Innenraum-Luftstroms aus einem begrenzten Innenraumbereich,

(b) Vermischen des Innenraum-Luftstroms aus (a) mit 0.1 bis 1500 ppm Ozon,

(c) Behandeln der Mischung aus Schritt (b) in einer Luftbehandlungszone, die einen Oxidationskatalysator auf einem festen Träger enthält, der ein Metall umfaßt, welches ausgewählt ist aus Eisen, Ruthenium, Osmium, Kobalt, Rhodium, Iridium, Nickel, Palladium, Platin, Molybdän und Wolfram,

(d) Behandeln der behandelten Mischung aus Schritt (c) in einer Ozonzersetzungszone, die einen Ozonzersetzungskatalysator enthält, ausgewählt aus nichtzeolithischen Molekularsieben, Aktivkohle und einem Katalysator auf einem Träger, welcher ein Metall umfaßt, ausgewählt aus Nickel, Palladium, Platin, Mangan, Kupfer und Silber, um einen behandelten Luftstrom zu erhalten, der nicht mehr als 0.05 ppm Ozon enthält, und

(e) Rückführen des behandelten Luftstroms aus Schritt (d) in den begrenzten Innenraumbereich von Schritt (a).

**2.** Verfahren gemäß Anspruch 1, worin das Metall des in Schritt (c) verwendeten Oxidationskatalysators auf einem Träger aus Molekularsieb des Zeolith- oder Nichtzeolith-Typs aufgebracht ist.

**3.** Verfahren gemäß irgendeinem der Ansprüche 1 und 2, worin der in Schritt (d) verwendete Ozonzersetzungskatalysator ein Metallkatalysator auf einem Träger ist.

**4.** Verfahren gemäß Anspruch 3, worin der Träger des Ozonzersetzungskatalysators ein poröser Träger ist.

**5.** Verfahren nach mindestens einem der Ansprüche 1 bis 4, worin die Temperatur und der Druck der Luftbehandlung im wesentlichen bei Umgebungsbedingungen liegen.

**6.** Verfahren gemäß mindestens einem der Ansprüche 1 bis 5, welches durchgeführt wird bei den Bedingungen der relativen Luftfeuchtigkeit der Umgebung, der Umgebungstemperatur und des Umgebungsdrucks des der Behandlung unterzogenen Luftstroms.

**7.** Apparat, der eine katalytische Zone zur Verunreinigungsentfernung umfaßt, welche eine Zone zur katalytischen Behandlung von Ozon umfaßt und eine Zone zur katalytischen Zerstörung von Ozon, der angeordnet ist in Verbindung mit der Luftzirkulation in einer großen begrenzten Struktur, in dem die Zone zur katalytischen Entfernung von Verunreinigungen enthält:

a) ein Transportmittel zum Leiten eines Luftstroms aus der großen begrenzten Struktur zur Zone der katalytischen Entfernung von Verunreinigungen,

b) ein Transportmittel zum Leiten des Luftstroms von der Zone der katalytischen Entfernung von Verunreinigungen zur großen begrenzten Struktur,

c) eine Zone zur katalytischen Ozonbehandlung, umfassend

i) eine Quelle für Ozon, verbunden mit der Zone der katalytischen Ozonbehandlung,

ii) eine offene Verbindung zum Transportmittel für das Leiten von Luft aus der großen begrenzten Struktur zur Zone katalytischer Ozonbehandlung,

iii) ein Verteilungsmittel zum Vermischen des Ozons mit der aus der großen begrenzten Struktur in die Zone katalytischer Ozonbehandlung eingeleiteten Luft,

iv) eine feste poröse Oxidationskatalysator-Schicht, in Strömungsrichtung nach dem Verteilungsmittel angeordnet, zu dem die ozonhaltige Luft vom Verteilungsmittel geleitet wird, wobei der Oxidationskatalysator ein auf einen Träger aufgebrachter Katalysator ist, welcher ein Metall umfaßt, das ausgewählt ist aus Eisen, Ruthenium, Osmium, Kobalt, Rhodium, Iridium, Nickel, Palladium, Platin, Molybdän und Wolfram,

v) einen Auslaß aus der Zone katalytischer Ozonbehandlung zu einer Zone katalytischer Ozonzerstörung,

d) eine Zone katalytischer Ozonzerstörung, welche umfaßt

i) eine offene Verbindung zu der Zone katalytischer Ozonbehandlung,

ii) einen festen porösen Ozonzerstörungs-Katalysator, der geeignet angeordnet ist, um in Kontakt mit dem aus der Zone katalytischer Ozonbehandlung zur Zone katalytischer Ozonzerstörung geleiteten Luftstrom zu treten, wobei der Ozonzerstörungskatalysator ausgewählt ist aus nichtzeolithischen Molekularsieben, Aktivkohle und einem Katalysator auf einem Träger, der ein Metall umfaßt, das ausgewählt ist aus Nickel, Palladium, Platin, Mangan, Kupfer und Silber,

iii) einem Auslaß aus der Zone katalytischer Ozonzerstörung zu dem Transportmittel zum Leiten des Luftstroms von der Zone zur katalytischen Entfernung von Verunreinigungen zu der großen begrenzten Struktur,

e) Ozonkontrollgeräte, die angeordnet sind, um die Konzentration des Ozons im Luftstrom festzustellen, der zur Zone katalytischer Verunreinigungsentfernung geleitet und aus dieser abgeleitet wird, und Mittel zur Regulierung des Ozons, mit dem die Zone katalytischer Verunreinigungsentfernung versorgt und das aus dieser abgeleitet wird.

**8.** Apparat gemäß Anspruch 7, worin der Apparat für die katalytische Entfernung von Verunreinigungen verbunden ist mit einem oder mehreren der Klimatisierungs- und Beheizungs-Systeme des großen begrenzten Bereichs und zusammen mit solchen Systemen betrieben wird.

**9.** Apparat gemäß mindestens einem der Ansprüche 7 bis 8, worin die Druckluftgebläse der Heizung oder die Klimatisierungs-Systeme die Luftströmungsgeschwindigkeit durch die Zone katalytischer Entfernung von Verunreinigungen regeln.

**10.** Apparat gemäß mindestens einem der Ansprüche 7 bis 9, worin sich die Zone zur katalytischen Entfernung von Verunreinigungen auf der Seite der rückgeführten Luft oder auf der Druckluftseite der Heizungen und/oder der Klimatisierungs-Ausrüstung befindet, die den großen begrenzten Bereich versorgt, und Teil der Luftzirkulationsanordnung des großen begrenzten Bereichs ist.

**11.** Apparat gemäß mindestens einem der Ansprüche 7 bis 9, worin die Zone zur katalytischen Entfernung von Verunreinigungen auf der Seite der zurückgeführten Luft des Klimatisierungs- und/oder Beheizungssystems befindet, mit dem sie verbunden ist.

**12.** Apparat gemäß mindestens einem der Ansprüche 7 bis 11, worin ein Filtrationsmittel zur Entfernung von Partikeln aus dem Luftstrom vor oder nach dem Ozonverteilungsmittel, das zum Vermischen des Ozons mit der Luft verwendet wird, angeordnet ist und das sich vor festen porösen Oxidationskatalysatorschicht befindet, die in Strömungsrichtung nach dem Verteilungsmittel liegt.

## Revendications

**1.** Procédé pour traiter de l'air confiné contenant des polluants comprenant des composés organiques volatils et des polluants biologiques comprenant les étapes suivantes :

(a) on soutire un flux d'air confiné à partir d'une enceinte confinée;

(b) on mélange le flux d'air confiné issu de l'étape (a) avec 0,1 à 1500 ppm d'ozone;

(c) on traite le mélange issu de l'étape (b) dans une zone de traitement de l'air contenant un catalyseur d'oxydation solide supporté qui comprend un métal choisi parmi le fer, le ruthénium, l'osmium, le cobalt, le rhodium, l'iridium, le nickel, le palladium, le platine, le molybdène et le tungstène;

(d) on traite le mélange issu du traitement de l'étape (c) dans une zone de décomposition de l'ozone contenant un catalyseur pour la décomposition de l'ozone choisi parmi les tamis moléculaires non zéolitiques, le charbon actif et un catalyseur supporté comprenant un métal choisi parmi le nickel, le palladium, le platine, le manganèse, le cuivre et l'argent de façon à obtenir un flux d'air traité ne contenant pas plus de 0,05 ppm d'ozone; et

(e) on fait circuler le flux d'air traité issu de l'étape (d) dans l'enceinte confinée de l'étape (a).

**2.** Procédé selon la revendication 1, dans lequel le métal du catalyseur d'oxydation utilisé à l'étape (c) est supporté sur un tamis moléculaire de type zéolitique ou nonzéolitique.

**3.** Procédé selon l'une quelconque des revendications 1 et 2, dans lequel le catalyseur pour la décomposition de l'ozone utilisé dans l'étape (d) est un catalyseur métallique supporté.

**4.** Procédé selon la revendication 3, dans lequel le support du catalyseur pour la décomposition de l'ozone est un support poreux.

**5.** Procédé suivant au moins une des revendications 1 à 4, dans lequel la température et la pression lors du traitement de l'air correspondent essentiellement aux conditions ambiantes.

**6.** Procédé selon une au moins des revendications 1 à 5, qui est mis en oeuvre dans des conditions d'humidité relative ambiante, de température ambiante et de pression ambiante pour le flux d'air soumis au traitement.

**7.** Dispositif comprenant une zone de dépollution catalytique comprenant une zone de traitement catalytique à l'ozone et une zone de destruction catalytique de l'ozone, implanté en association avec un circulateur d'air dans une structure confinée de grande dimension, dans lequel la zone de dépollution catalytique contient :

a) un moyen de transport pour faire passer un flux d'air de la structure confinée de grande dimension vers la zone de dépollution catalytique,

b) un moyen de transport pour le passage du flux d'air de la zone de dépollution catalytique vers la structure confinée de grande dimension,

c) une zone de traitement catalytique à l'ozone comprenant

(i) une source d'ozone associée à la zone de traitement catalytique à l'ozone,

(ii) une liaison ouverte au moyen de transport qui fait passer l'air de la structure confinée de grande dimension vers la zone de traitement catalytique à l'ozone,

(iii) un moyen de distribution pour mélanger l'ozone avec l'air de la structure confinée de grande dimension introduit dans la zone de traitement catalytique à l'ozone,

(iv) une couche de catalyseur d'oxydation solide poreux située en aval du moyen de distribution et dans laquelle on fait passer l'air contenant de l'ozone provenant du moyen de distribution, ledit catalyseur d'oxydation étant un catalyseur supporté comportant un métal choisi parmi le fer, le ruthénium, l'osmium, le cobalt, le rhodium, l'iridium, le nickel, le palladium, le platine, le molybdène et le tungstène,

(v) une prise de sortie allant de la zone de traitement catalytique à l'ozone à une zone de destruction catalytique de l'ozone,

d) une zone de destruction catalytique de l'ozone comprenant

(i) une liaison ouverte à la zone de traitement catalytique à l'ozone,

(ii) un catalyseur solide poreux pour la décomposition de l'ozone mis en place de façon appropriée pour être en contact avec le flux d'air qui passe de la zone de traitement catalytique à

l'ozone vers la zone de desdestruction catalytique de l'ozone, ledit catalyseur de décomposition de l'ozone étant choisi parmi les tamis moléculaires non zéolitiques, le charbon actif et un catalyseur supporté comprenant un métal choisi parmi le nickel, le palladium, le platine, le manganèse, le cuivre et l'argent,

(iii) une prise de sortie allant de la zone de destruction catalytique de l'ozone vers le moyen de transport qui fait passer le flux d'air de la zone de dépollution catalytique vers la structure confinée de grande dimension,

e) des moyens de contrôle de l'ozone mis en place pour déterminer la concentration de l'ozone présente dans le flux d'air introduit dans et issu de la zone de dépollution catalytique et des moyens de régulation de l'ozone introduite à l'entrée et récupérée à la sortie de la zone de dépollution catalytique.

**8.** Dispositif selon la revendication 7, dans lequel le dispositif de la zone de traitement catalytique est associé à un ou plusieurs systèmes de conditionnement ou de chauffage de l'air dans l'enceinte confinée de grande dimension et fonctionne de concert avec ces systèmes.

**9.** Dispositif selon au moins une des revendications 7 à 8, dans lequel les ventilateurs d'air forcé du four ou des systèmes de conditionnement d'air contrôlent le débit d'air à travers la zone de dépollution catalytique.

**10.** Dispositif selon au moins une des revendications 7 à 9, dans lequel la zone de dépollution catalytique est située sur le côté air de retour ou le côté air forcé desdits fours et/ou appareil de conditionnement d'air desservant l'enceinte confinée de grande dimension et faisant partie du circuit de circulation d'air dans l'enceinte confinée de grande dimension.

**11.** Dispositif selon au moins une des revendications 7 à 9, dans lequel la zone de dépollution catalytique est fournie sur le côté air de retour des systèmes de conditionnement et/ou de chauffage d'air auxquels elle est associée.

**12.** Dispositif selon au moins une des revendications 7 à 11, dans lequel un moyen de filtration pour éliminer des particules du flux d'air est situé avant ou après le moyen de distribution d'ozone utilisé pour mélanger l'ozone avec l'air, et situé avant la couche de catalyseur d'oxydation solide poreux placée en aval du moyen de distribution.

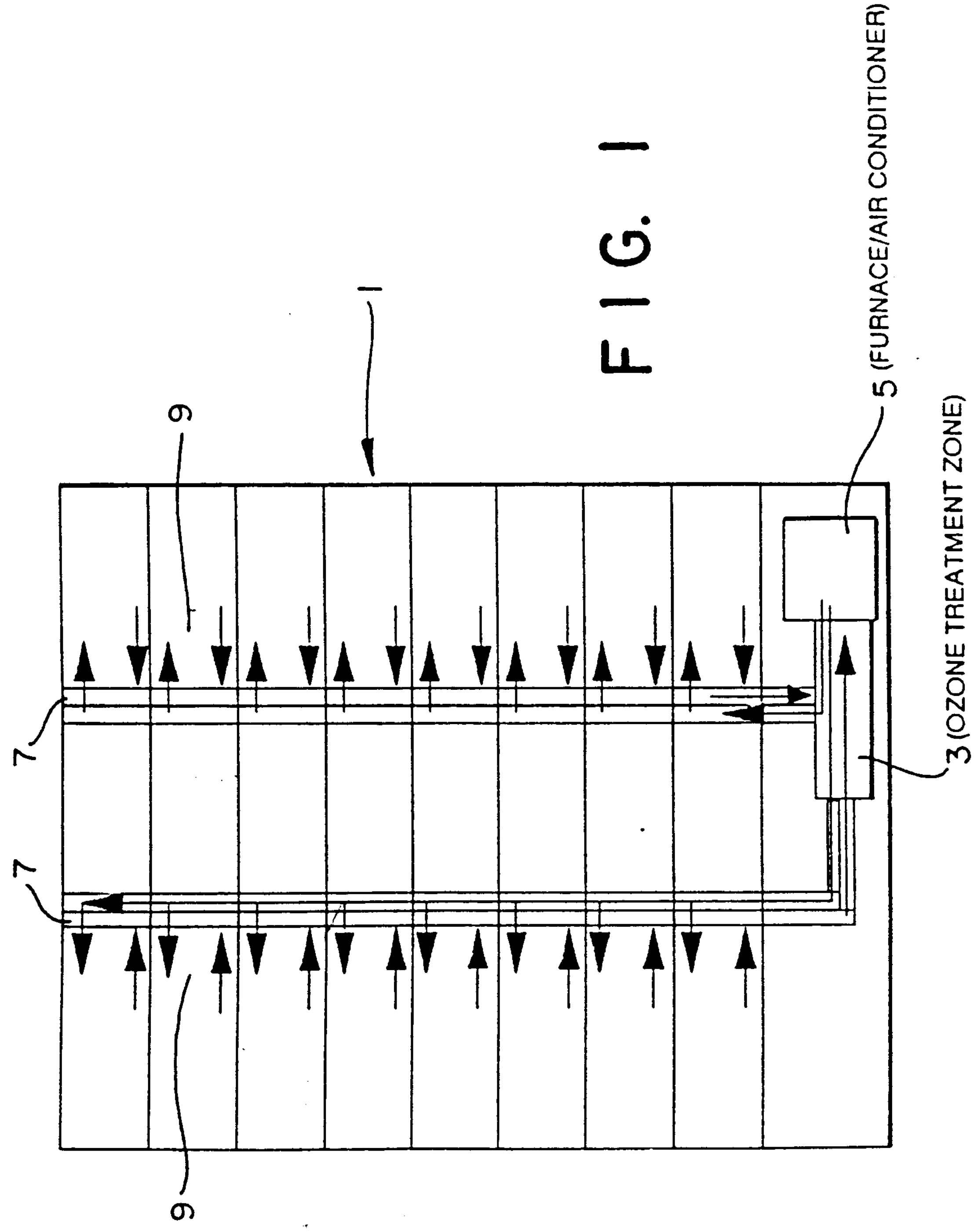


FIG. 1

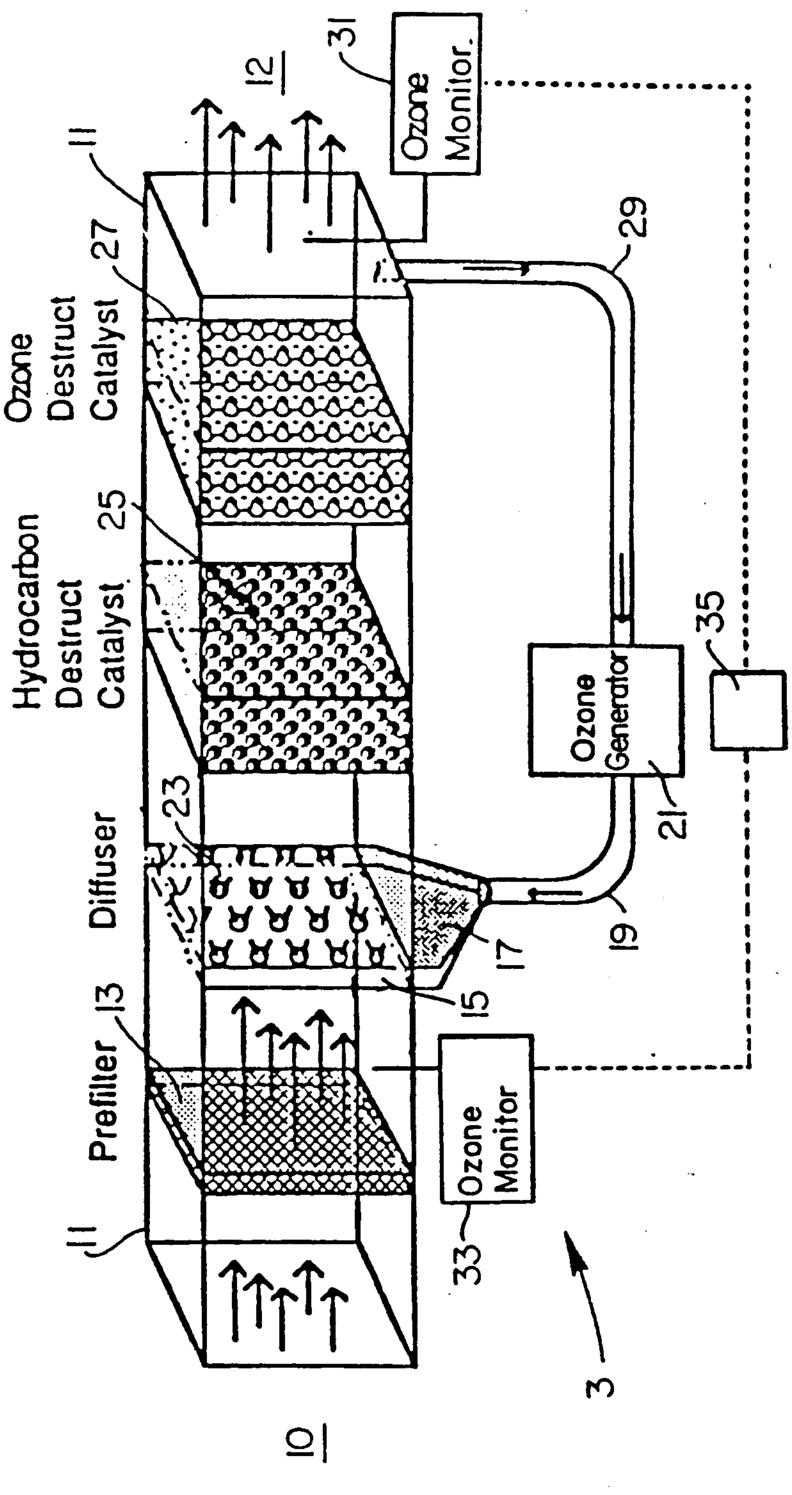
Prefilter
Diffuser
Hydrocarbon Destruct Catalyst
Ozone Destruct Catalyst
Ozone Generator
Ozone Monitor
Ozone Monitor.
10
11
12
13
15
17
19
21
23
25
27
29
31
33
35
3

FIG. 2

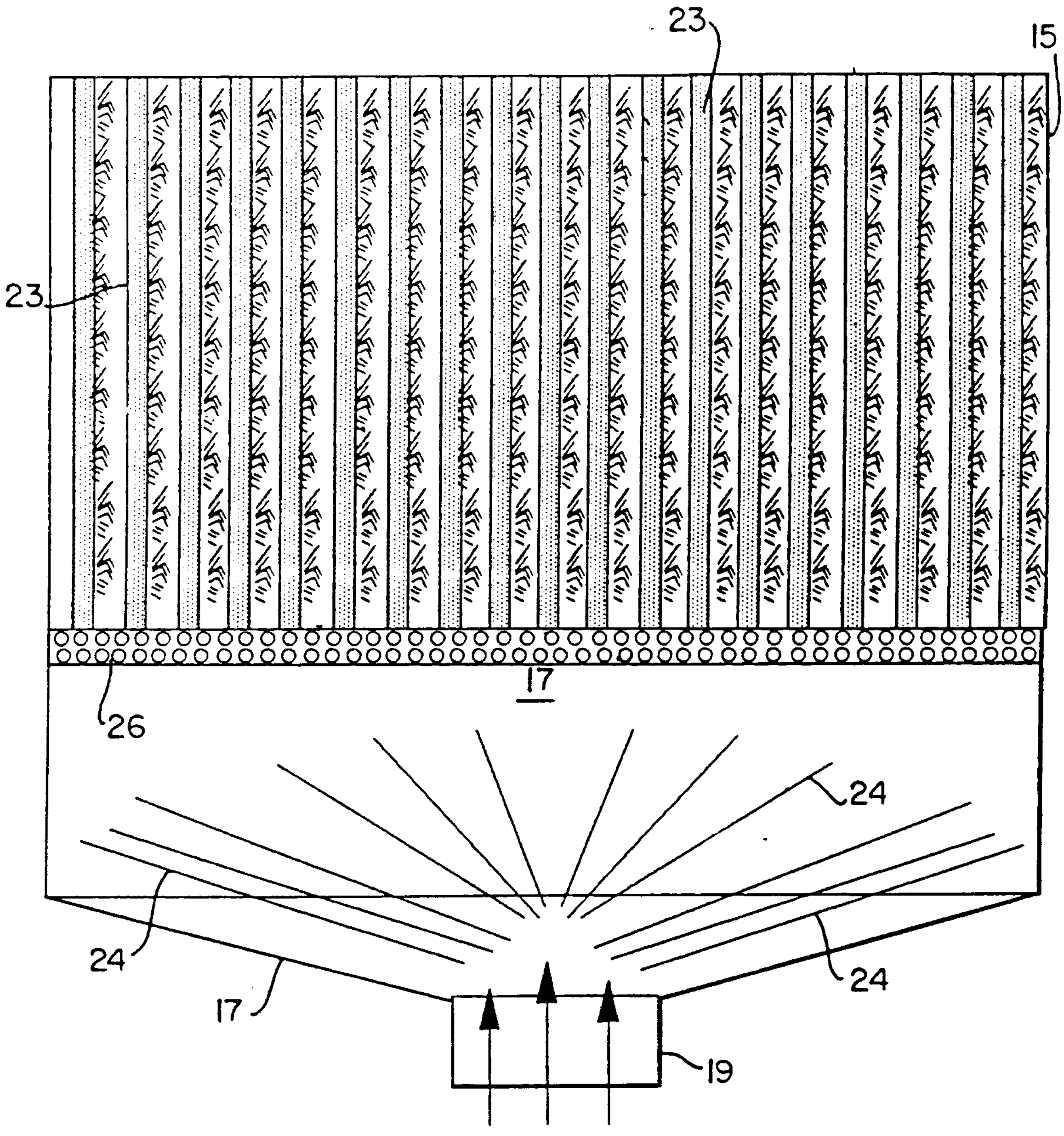


FIG. 3

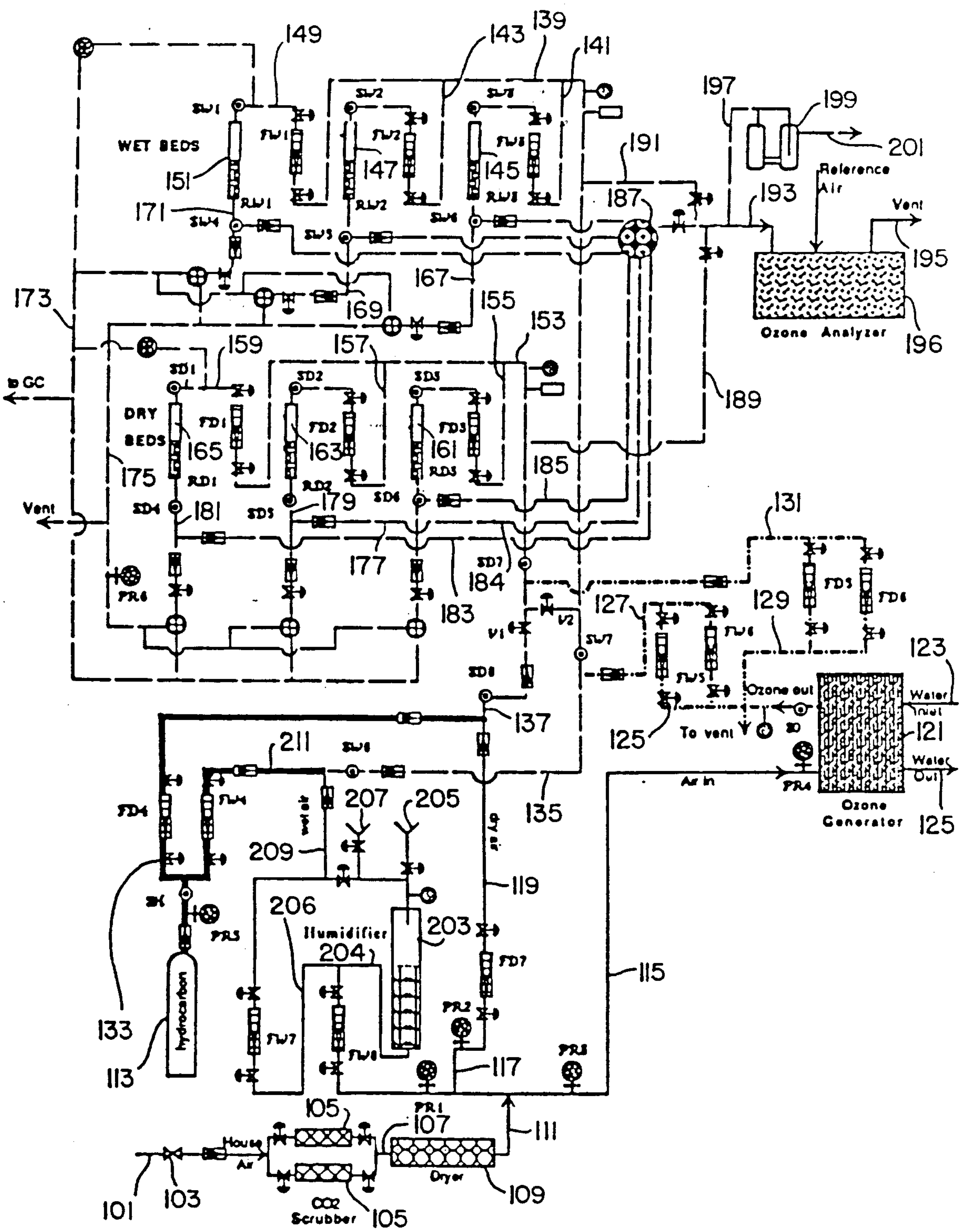
WET BEDS
DRY BEDS
to GC
Vent
Reference Air
Ozone Analyzer
Ozone out
To vent
Water inlet
Water Out
Air in
Ozone Generator
wet air
dry air
Humidifier
hydrocarbon
House Air
CO2 Scrubber
Dryer

F I G. 4